# EUROPEAN PATENT SPECIFICATION

(11) **EP 0 892 856 B1**
(45) Date of publication and mention of the grant of the patent: **09.11.2005**
(21) Application number: 97914790.7
(22) Date of filing: 27.02.1997
(51) Int. Cl.: C12Q 1/68

(54) **A METHOD FOR THE AMPLIFICATION AND DETECTION OF A NUCLEIC ACID FRAGMENT OF INTEREST**
METHODE ZUR VERVIELFÄLTIGUNG UND ZUM NACHWEIS EINES GEWÜNSCHTEN NUKLEINSÄUREFRAGMENTS
PROCEDE D'AMPLIFICATION ET DE DETECTION D'UN FRAGMENT D'ACIDE NUCLEIQUE D'INTERET

(30) Priority: 01.03.1996 US 12636 P
(43) Date of publication of application: 27.01.1999
(73) Proprietor: E.I. DU PONT DE NEMOURS AND COMPANY, Wilmington, Delaware 19898 (US)
(72) Inventor: EBERSOLE, Richard, C., Wilmington, DE 19810 (US); HENDRICKSON, Edwin, R., Hockessin, DE 19707 (US); FITZPATRICK-MCELLIGOTT, Sandra, Rose Valley, PA 19063 (US); PERRY, Michael, P., Landenberg, PA 19350 (US)
(74) Representative: Carpmaels & Ransford
(86) International application number: PCT/US1997/002892
(87) International publication number: WO 1997/032044

(56) References cited:
- EP-A- 0 587 298
- EP-A- 0 628 568
- EP-A- 0 745 690
- WO-A-93/10267
- WO-A-94/02634
- WO-A-95/13399
- WO-A-97/07235

### SEQUENCE LISTING

(1) GENERAL INFORMATION:
   (i) APPLICANT:
      (A) NAME: E. I. DU PONT DE NEMOURS AND COMPANY
      (B) STREET: 1007 MARKET STREET
      (C) CITY: WILMINGTON
      (D) STATE: DELAWARE
      (E) COUNTRY: UNITED STATES OF AMERICA
      (F) POSTAL CODE (ZIP): 19898
      (G) TELEPHONE: 302-992-4931
      (H) TELEFAX: 302-773-0164
      (I) TELEX: 6717325
   (ii) TITLE OF INVENTION: METHOD FOR THE AMPLIFICATION AND DETECTION OF NUCLEIC ACID FRAGMENTS
   (iii) NUMBER OF SEQUENCES: 16
   (iv) COMPUTER READABLE FORM:
      (A) MEDIUM TYPE: DISKETTE, 3.50 INCH
      (B) COMPUTER: IBM PC COMPATIBLE
      (C) OPERATING SYSTEM: MICROSOFT WINDOWS 95
      (D) SOFTWARE: MICROSOFT WORD 7.0
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 60/012,636
      (B) FILING DATE: MARCH 1, 1996
   (viii) ATTORNEY/AGENT INFORMATION:
      (A) NAME: SIEGELL, BARBARA C.
      (B) REGISTRATION NUMBER: 30,684
      (C) REFERENCE/DOCKET NUMBER: CR-9806
(2) INFORMATION FOR SEQ ID NO:1:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
   GGCACGACGG TTATGTTAGA C 21
(2) INFORMATION FOR SEQ ID NO:2:
   (i) SEQUENCE CHARACTERISTICS:
      (A) LENGTH: 58 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
   NATGTACTCT ACTCTCTCTG CTTTCCCATC TANGCGTTAA TTATGAACTC TAGTTTNA 58
(2) INFORMATION FOR SEQ ID NO:3:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
   GCGGCTTGCC CTGGAGATTG AAATACGTGA TGCAAAGTAG GAAGCTATAT AAGTTAATAG 60
   GAATCGTCAA AGCATGGCGC ACAC 84
(2) INFORMATION FOR SEQ ID NO:4:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii). MOLECULE TYPE: DNA (genomic)
   (xi) SEQUENCE DESCRIPTION: SEQ ID NO:4:
   GCGGCTTGCC CTGGAGATTG A 21
(2) INFORMATION FOR SEQ ID NO:5:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
   GTGTGCGCCA TGCTTTGACG A 21
(2) INFORMATION FOR SEQ ID NO:6:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:6:
   NAATACGTGA TGCNAAGTAG GAAGCTATAT AAGTTAATAG GNA 43
(2) INFORMATION FOR SEQ ID NO:7:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
   TTATGCCATT N 11
(2) INFORMATION FOR SEQ ID NO:8:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:8:
   SAATGGCATA ACAGGATAAC AATAATCAAA TAAAAGTTTT AAACAAATAA 50
(2) INFORMATION FOR SEQ ID NO:9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:9:
   GTGTCACTCC ATACATCTCG 20
(2) INFORMATION FOR SEQ ID NO:10:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:10:
   AACTGCTGTC CACTTCTGAG 20
(2) INFORMATION FOR SEQ ID NO:11:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
   CATACCTTCT GGTGCTAGAG 20
(2) INFORMATION FOR SEQ ID NO:12:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:12:
   NTAATCCTGT AGGCAGAGAA CTCTANACTC ATCCCCCAGA AA 42
(2) INFORMATION FOR SEQ ID NO:13:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:13:
   TAATCCTGTA GGCAGAGAAC TCTANACTCA TCCCCCAGAN A 41
(2) INFORMATION FOR SEQ ID NO:14:
   (i) SEQUENCE CHARACTERISTICS:
      (A) LENGTH: 97 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:14:
   GCGAGGATGG CGAACAACAA GAATGTACTC TACTCTCTCT GCTTTCCCAT CTATGCGTTA 60
   ATTATGAACT CTAGTTTACC ACACCCATTC CGCCCGA 97
(2) INFORMATION FOR SEQ ID NO:15:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:15:
   GCGAGGATGG CGAACAACAA GA 22
(2) INFORMATION FOR SEQ ID NO:16:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:16:
   TCGGGCGGAA TGGGTGTGGT 20

## Description

### FIELD OF INVENTION

The present invention relates to the field of molecular biology and to methods for nucleic acid replication and detection. More specifically, the invention describes a method for the detection of amplified nucleic acid fragments of interest that incorporates a ligand labeled probe into an amplification reaction. The probe is present throughout the amplification reaction but is non-participatory. In one embodiment, the probe anneals to the replicated analyte and serves as a capture and immobilization reagent for the detection of the nucleic acid fragment of interest.

### BACKGROUND

The ability to detect and quantitate specific nucleic acid fragments is becoming increasingly important in the fields of medical, agricultural, food and environmental diagnostics. Accordingly, a multiplicity of methods has been developed to identify gene sequences that are unique to a disease causative agent, a bacterial contaminant or will predict an adult plant phenotype.

Low concentrations of many of these nucleic acid sequences require most methods to first amplify the sequence of interest using common nucleic acid amplification protocols such as but not limited to polymerase chain reaction (PCR, Mullis et al., U.S. Patent 4,683,202), ligase chain reaction (LCR, Tabor, S. et al.. *Proc. Acad. Sci.* USA 82, 1074, (1985)) or strand displacement amplification (SDA. Walker, et al., *Proc. Natl. Acad Sci. U.S.A.,* 89. 392, (1992)). These methods use nucleic acid primers to logarithmically amplify a very specific nucleic acid sequence fragment from a diverse background of non-specific sequences.

Amplification of the nucleic acid sequence of interest serves to increase its concentration in the sample. Detection of the sequence is effected through the incorporation of a label. Methods of immobilizing specific a nucleic acid sequence for detection often involve capture by hybridization of the sequence fragment to a probe of complementary sequence (Corti, A., et al., *Nuc. Acids Res.,* 19, 1351 (1991)). Alternatively the nucleic acid sequence of interest may be labeled with a particular immuno or affinity reactive ligand, where immobilization is effected by the interaction of the ligand with its reactive counter part. Examples of immuno-reactive ligands pairs are various antigen - antibody pairs and an example of affinity reactive pair would be a biotin - streptavidin protein complex.

Thus, the elements for effective detection of a nucleic acid sequence of interest may involve (i) the replication of the sequence fragment by a method of nucleic acid replication such as PCR, (ii) annealing of the probe of complementary sequence and (iii) immobilization of a hybridized probe/fragment complex and (iv) the detection of immobilized fragment.

Methods incorporating these elements are known. For example, WO/9508644 discloses a method for the detection and quantification of nucleic acids where amplification generates single-stranded nucleic acid fragments that are then hybridized to an immobilized probe during a hybridization step and reported by an electrochemiluminescent label. Additionally, McMahon et al., (U.S. 5310650) teach the capture of an amplified nucleic acid fragment by solution phase hybridization to a probe immobilized on a chromatographic membrane. WO95/13399 and EP-A2-0745690 disclose detection of an amplified nucleic acid using probes having little or no signal of their own but which undergo a conformational change to produce a detectable signal when hybridized to a target.

Although methods such as these are useful, they require a separate hybridization procedure to immobilize and detect the nucleic acid sequence of interest. A more efficient method would permit the detection probe to be placed in the sample before amplification, and then remain in the sample during the amplification reaction where it would function to anneal to at least one strand of the amplified fragment after termination of the reaction. In such a method the probe should not participate in the replication steps of the amplification reaction, yet it should be sufficiently resistant to degradative effects of amplification so as not limit detection.

The problem to be overcome, therefore, is to develop a method for the immobilization and detection of a specific nucleic acid sequence fragment of interest using a non-participatory probe that will hybridize to the amplified fragment, but cannot be extended at the 3' end and will not be significantly degraded by amplification reagents such as the polymerase.

Primers or probes may be rendered non-participatory in a nucleic acid amplification protocol through the introduction of replication blocking moiety on the 3' terminus. Fragments containing 3' chain terminating dideoxy groups were first reported by Sanger et al., (Proc. Natl. Acad. Sci. USA, 74, 5463, (1977)) in a method for DNA sequencing. Since then, 3' blocked primers have been developed to be used in methods for target nucleic acid amplification to modulate or inhibit the amplification reaction. For example W0/9403472 discloses a method for amplifying a target DNA molecule under conditions of constant temperature using a mixture of 3' blocked and unblocked primers. The function of the 3' blocked primer is to enhance the efficiency of specific amplification and reduce background. The method of WO/9403472 does not use the 3' blocked primer as a detection probe.

Similarly U.S. 5169766 teaches a method for amplifying a nucleic acid molecule using a single-stranded sequence containing a T7 promoter. The 3' end of the sequence is complementary to the target to be amplified, but is also blocked with a 3' terminal nucleotide lacking a 3' hydroxyl group. The blocked sequence is incapable of serving as a substrate for DNA chain extension reactions. When the blocked sequence hybridizes to a linear target having a 3' hydroxyl terminus, the 3' terminus can then be extended producing a sequence complementary to that of the blocked sequence. The blocked sequence is useful for modulating the amplification reaction, but is not used as a probe for the detection of a specific nucleic acid molecule.

There are a few examples in the prior art of methods of detecting DNA amplified by PCR that use immobilized capture probes that are blocked at the 3' terminal and present in the amplification reaction EP-A2-0628568 describes the immobilization of amplified nucleic acids by an immobilized capture probe that is present in a DNA amplification reaction and is protected at the 3' end from extension by the addition of a deoxy or dideoxy terminator group. WO 93/10267 describes a method for the detection of amplified DNA using an immobilized detection probe having an electrochemiluminescent reporter moiety at the 3' end of the probe. WO 97/07235 describes a method for detecting a target nucleic acid using an immobilized hybridization probe having a modified 3' end such that the probe is non-extendible and is present in the amplification reaction. None of these documents discloses a homogeneous method for detection of an amplified nucleic and analyte with prior immobilisation of the amplified analyte. Furthermore, one of these documents disclose the use of cordycepin as a terminator group. The use of cordycepin as a terminator for a probe in a a method for performing an LCR reaction is described in WO 94/24311.

One of the difficulties in using 3' blocked primers and probes in nucleic acid amplification protocols is the tendency for the polymerase to degrade the molecule from the 5' end. DNA polymerases are known to possess a 5' to 3' polymerase dependent exonuclease activity. These include E. coli Polymerase I and T. aquaticus (Taq) DNA polymerase. (Cozzarelli, N. R., Kelly, R. B. and Komberg, A. *J. Mol. Biol.* 45,513 (1969); (Longley et al., *(Nuc. Acids. Res.,* 18, 7317, (1990). Taq DNA polymerase has been shown to degrade an oligonucleotide that is annealed to a template and is downstream from a primer being chain extended (Longley et al., *(Nuc. Acids. Res.,* 18, 7317, (1990)). However, (Lewis et al., *Nuc. Acids. Res.,* 22, 2859, (1994)) have shown that oligonucleotides, which can be extended by Taq DNA polymerase and are hybridized to a sequence that is between two flanking amplification primers, can block PCR amplification of the intended full-length sequence fragment. This results, instead, in the amplification of the nested fragment. Furthermore, oligonucleotides that are mismatched or modified at their 3'- termini to prevent chain extension are not able to block PCR amplification of the full-length fragment and therefore, are probably displaced.

Strand-displacement is the process of removing a complementary strand or oligonucleotide that is located 3' to a primer being extended by DNA polymerase. This property has been observed in 5' to 3' exonuclease deficient E. coli DNA polymerase I (Klenow fragment) (Walker et al., *Proc. Natl. Acad Sci. U.S.A.*, 89, 392, (1992)). Strand-displacement has also been observed with Taq DNA polymerase. Oligonucleotides modified at their 5'-termini or composed of exonuclease-resistant phosphorothioate nucleotide linkages were unable to prevent the progress of Taq DNA polymerase, suggesting a strand-displacement activity for Taq DNA polymerase.

Applicants have developed a detection probe system for immobilization and detection of a polymerase replicated nucleic acid fragment. The detection probe is added to the assay at the beginning of the replication reaction and hybridizes to at least one strand of an amplified sequence to form a probe/replicated-strand hybrid complex, but it is not extended during the replication procedure. The probe is blocked at the 3' end with a replication blocking moiety which prevents 3' extension of the probe. To allow for hybridization, the probe is designed to be complementary in sequence to either strand of the replicated analyte sequence. Further, the probe may be labeled with a member of a binding pair. The probe/replicated-strand hybrid complex maybe immbolized and reported by either or both of these labeling methods. Surprisingly, three unexpected observations from the this probe detection system were made:
(i) the product yield in a replication (amplification) reaction is not significantly decreased by the presence of the detection probe.
(ii) the hybridized detection probe is not significantly displaced from the probe/replicated strand hybrid complex by the complementary nucleic acid strand of the replicated target to interfere with detection and
(iii) the detection probe is not significantly degraded at the 5' end during the reaction by the polymerase's 5' to 3' exonuclease activity to prevent detection.

Wilson et al., (*J. Clin. Microbiol,* 31, 776, (1993); Qiao et al., (*Parasitology*, 110 269, (1995)) and Aguirre et al., (*Transactions of the Royal Society of Tropical Medicine and Hygiene*, 89, 187 (1995)) describe a method to detect PCR products that combines probe hybridization with an enzyme-linked immunosorbent assay (ELISA). The method is termed PCR-solution hybridization enzyme-linked immunoassay (PCR-SHELA). This method enables the PCR product to be hybridized to a labeled probe, which is then immobilized onto a microtiter plate and detected colorimetrically. Although both groups report using PCR-SHELA as a method to detect pathogens. Both approaches differ in assay configurations, primer and probe melting temperatures. (Tm's and base composition) and in the stage of the assay of which the probe is added.

The design of the probe and PCR conditions, as described by Qiao et al., (1995), allowed for the addition of the probe at the beginning of the PCR reaction. More specifically, since the annealing temperature of the probe was lower than the annealing temperature maintained during the PCR reaction, the primers were able to anneal to the target whereas the probe was not. Amplification, therefore, would not be impeded by the possible presence of an annealed probe. However, Aguirre et al., (1995) report that, because the annealing temperature of their probes was the same as that of the primers, the probes were added to an aliquot of the product after PCR cycling was complete. In addition, the probes used in both the Qiao and Aguirre studies contained 3' OH group and were, therefore, potentially replication competent, even though the probe used in the Qiao experiments contained a 3' mismatched poly T tail.

While Mayrand et al. (WO 96/34983) describe a hybridization probe technology for the detection of amplified DNA in a single reaction vessel by the addition of a single reagent, they are limited in the application of their technology. One such limitation is the use of a fluorescent/quenching probe system. The system comprises a probe that contains a fluorescing molecule at one end and a quenching molecule at the other. In its single stranded form, in absence of target, the probe exists as a flexible random coil bringing the fluorophores in close proximity to one another resulting in signal quenching. However, when the probe hybridizes to its target nucleic acid, the fluorophores are separated from each other thus generating a detectable signal. The Applicant's detection probe technology, on the other hand, is not limited to a flourescence/quenching system. A number of reporter ligands and detection formats can be used with the Applicant's technology.

Furthermore, as described in Mayrand et al, there are constraints placed on the probe composition. Since the probe cannot hybridize during the polymerization step, the size and melting temperature (Tm) of the probe are limited. The probe must be smaller or have a lower Tm than the primers so that it does not anneal during polymerization unless a probe displacer (e.g. helicase) is used. Even more restrictive is the preferred use of a displacer inactivation step. The detection system is even further constrained by the use of an exonuclease negative polymerase or probes resistant to the exonuclease digestion. Also, since a separate probe hybridization step is required after the completion of the PCR cycling, the technology would not be applicable to a real-time monitoring of PCR product formation. In contrast, the Applicant's detection method, described herein, includes the use of the probe in a homogeneous detection probe system (HDPS) which allows for real-time monitoring of product formation.

The Applicant's method, is not restricted by base composition of the probes and primers, Tm's, annealing temperatures, nor PCR cycling conditions. Applicant's method uses replication inhibited probes of varying length and allow sequence specific probe composition applicable to most targets. Most significantly, the applicants' method does not prevent the probe from annealing during PCR. Unexpectedly, amplification and detection of the target sequence are not inhibited with longer probes. Even though low, standard annealing temperatures are used in the Applicants' method, the probes do not inhibit replication nor decrease product yield.

Applicants' method amounts to an improvement in the art by introducing the detection probe at the beginning of the replication (amplification) reaction. The approach addresses two problems: (i) it eliminates the need for an additional, time-consuming hybridization procedure, resulting in a faster and more efficient assay. And, (ii) it addresses the problem of the detection of non-specific secondary sequences in the sample. The detection of secondary sequences is one of the major problems with the amplification (replication) methods (e.g., PCR, LGR, SDA). These unwanted replication products are produced from primer-dimer interaction and from secondary primer sequence sites in the nucleic acid sample. The probe can be designed with sequences that are specific for the internal sequences that are between the flanking replication (amplification) primer sequences: Therefore, this method reduces detection of unwanted sequence contamination (assay noise).

Further, the Applicant's method obviates the need for electrophoresis and allows for the detection of DNA products in several formats. These can include colorimetric detection or luminescent detection (e.g. bioluminescence and chemiluminescence) on both membranes and microtiter plates. Detection of replicated (amplified) nucleic acids are improved by the use of these sequence specific probes. Moreover, with the use of probe sequences specific for different nucleic acid analytes and the use of different binding pairs (ligands) as capture reagents, multiple target detection is possible.

### SUMMARY OF THE INVENTION

The present invention provides a method for the detection of a target nucleic acid analyte sequence in a nucleic acid replication reaction, comprising the steps of:
(i) contacting at least one target nucleic acid sequence with a nucleic acid replication composition containing a homogeneous detection probe system conprising at least one pair of probes said pair consisting of:
   (a) a first, signal generating probe comprising a first member of a reporter pair, a target domain, a first probe binding domain and replication inhibitor moiety wherein said inhibitor moiety is a 3' deoxynucleotide, and;
   (b) a second, signal modifying probe comprising a second member of a reporter pair and a second probe binding domain complementary to said first probe binding domain wherein the first and second members of the reporter pair are capable of reacting with each other to produce a detectable signal;
(ii) replicating the target nucleic acid sequence in the replication composition of step (i) to produce a nucleic acid analyte and under reaction conditions that permit the formation of an analyte/probe hybrid wherein said hybrid consists of said target analyte nucleic acid and the signal generating detection probe; and
(iii) detecting the presence said analyte/probe hybrid.

The present method is especially suited for analysis of replication products produced by primer-directed amplification procedures (PCR, LCR, SDA), but also can be used with systems that use other replication initiation sequences such as, RNA replication procedures (replicative RNA systems (Qβ), DNA-dependent RNA polymerase promoter systems (T7 or SP6) or primer-directed replication/RNA promoters combination amplification systems (NASBA-Kievits et al., *J. Virol* Methods 35,273 (1991))). The instant process is an improvement over known replication - product detection methods because the non-participatory detection probe allows for the replication of the target-nucleic acid and hybridization of the detection probe in one procedural step, before it is immobilized and detected. This removes the multiple handling steps required for hybridization of the probe to the replicated strand. Thus, this invention provides a detection method in which a replication reaction occurs concurrently with the hybridization of the non-participatory detection probe to the replication product in the sample.

The invention further provides a method of detection where a specific fragment must be distinguished from a large background of nonspecific products, as would result from degenerate PCR amplification, for example. Typically in these cases, the products, after gel electrophoresis, are transferred to membranes and hybridized with a labeled, sequence specific probe. However, a method using the instant detection probe technique, where the probe is already hybridized to the final product after cycling is complete, the signal can be developed directly in the gel through the addition of the antibody conjugate and chromogenic substrate (Sun, et al., *BioTechniques,* 16, 782, (1994)). The specific fragment can therefore be identified rapidly without time-consuming membrane transfer and overnight hybridization procedures.

It is anticipated that the invention can also be applied to *in situ* PCR. *In situ* PCR involves localized amplification of nucleic acid directly in the cell. The cells may be in suspension, in tissue sections or slices, or adhered to tissue culture plates. An in-depth discussion of *in situ* PCR is given in Nuovo et al., *PCR in situ Hybridization,* Raven Press, 214-306 (1994). Typically, a reactive label such as digoxigenin is incorporated into the PCR product and the product is then detected by a reporter antibody and substrate. Alternatively, the detection probe containing fluorescein, for example, could be added at the beginning of the PCR procedure. The resultant, localized PCR product/probe hybrid would cause the cells to fluoresce. The fluorescent positive cells could then be detected by a variety of methods, including flow cytometry, for example (Gibellini et al.. *Analytical Biochemistry,* 228, 252-258, (1995)). With the detection pmbe technology, the incubation steps with the reporter antibody and substrate associated with traditional *in situ* PCR would, therefore, be eliminated.

### BRIEF DESCRIPTION OF THE FIGURES

Figure la is a diagram of the reverse transcription (RT) product used as a target nucleic acid and the relative positions of the outer primers. 1156 and 1862, and the nested primers, 1281 and 1569.
Figure 1b is a diagram showing a 707 bp fragment target nucleic acid and the relative positions of the primers. *1281*, and *1569*, and the detection probe.
Figure 2 shows agarose gel and lateral flow detection of the nested 289 bp product amplified with and without the biotinylated detection probe.
Figure 3 is a graphic representation showing the effect of probe concentration on PCR product yield.
Figure 4a is a plot of mean pixel density vs. probe concentration using the 707 bp target showing the effect of probe concentration and template copy number on lateral flow detection.
Figure 4b is a plot of optical density vs. probe concentration using the 707 bp target showing the effect of probe concentration and template copy number on microtiter plate detection.
Figure 5 is a plot comparing the detection of amplified nucleic acid products by gel electrophoresis, lateral flow, and microtiter plate assays.
Figure 6 is a plot comparing the detection sensitivity of the reporter using chromogenic and chemiluminescent substrates in a microtiter plate assay.
Figure 7 is a plot comparing detection with the probe added before or after PCR amplification.
Figure 8 is a diagram of the additional PCR products produced in the presence of a non-terminated detection probe.
Figure 9 is a diagram representing multianalyte detection of two different nucleic acid analytes in the lateral flow format. Each analyte-specific probe contains a different capture moiety.
Figure 10 shows the results of two targets amplified in a multiplex fashion and detected by the lateral flow procedure.
Figure 11 shows that the detection probe technology can be successfully applied to the inununo-PCR procedure and that the signal generated on the membrane strips is representative of the amount of hCG present in the initial sample.

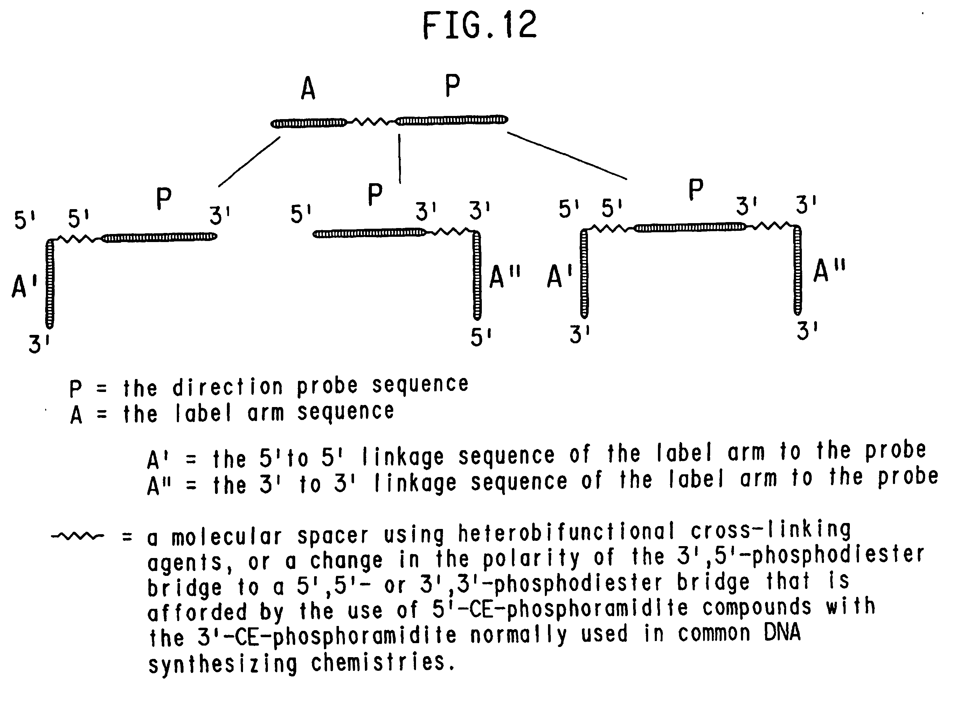

Figure 12 is graphic representation of the bivalent detection probe (BVDP). A BVDP is comprised of two oligonucleotide domains, a detection probe sequence (P) and label arm sequence (A). These two region are linked together by a molecular spacer.
Figure 13 is an illustration of the homogeneous detection probe system (HDPS) in the two-subunit format

### DETAILED DESCRIPTION OF THE INVENTION

As used herein, the following terms may be used for interpretation of the claims and specification.

The term "homogenous assay" means an assay for nucleic acid analyte wherein no separation of reactions is required for detection.

A "fragment" constitutes a fraction of the DNA sequence of the particular region. A "nucleic acid fragment of interest" refers to a fragment that is incorporated within or is part of a target nucleic acid sequence and is useful as a diagnostic element.

"Replication" is the process in which a complementary copy of a nucleic acid strand of the "target nucleic acid" is synthesized by a polymerase enzyme. In a "primer-directed" replication, this process requires a hydroxyl group (OH) at 3' position of (deoxy)ribose moiety of the terminal nucleotide of a "duplexed" "oligonucleotide" to initiate replication.

"Amplification" is the process in which replication is repeated in cyclic manner such that the number of copies of the "target nucleic acid" is increased in either a linear or logarithmic fashion.

The term "target nucleic acid" refers to the nucleic acid fragment targeted for replication (or amplification) and subsequent detection. Sources of target nucleic acids will typically be isolated from organisms and pathogens such as viruses and bacteria or from an individual or individuals, including but not limited to, for example, skin, plasma, serum, spinal fluid, lymph fluid, synovial fluid, urine, tears, blood cells, organs, tumors, and also to samples of in vitro cell culture constituents (including but not limited to conditioned medium resulting from the growth of cells in cell culture medium, recombinant cells and cell components). Additionally, it is contemplated that targets may also be from synthetic sources. Target nucleic acids are amplified via standard replication procedures to produce nucleic acid analytes.

The term "analyte" or "nucleic acid analyte" refers to a substance to be detected or assayed by the method of the present invention. Typical analytes may include nucleic acid fragments including DNA. RNA or synthetic analogs thereof.

The term "oligonucleotide" refers to primers, probes, oligomer fragments to be detected, labeled-replication blocking probes, oligomer controls, and shall be generic to polydeoxyribonucleotides (containing 2-deoxy-D-ribose), to polyribonucleotides (containing D-ribose) and to any polynucleotide which is an N glycoside of a purine or pyrimidine base (nucleotide), or modified purine or pyrimidine base. Also included in the definition of "oligonucleotide" are nucleic acid analogs (e.g., peptide nucleic acids) and those that have been structurally modified (e.g., phosphorothioate linkages). There is no intended distinction between the length of a "nucleic acid", "polynucleotide" or an "oligonucleotide".

Nucleotides are reacted to make oligonucleotides in such a fashion that the 5' phosphate of one nucleotide pentose ring is attached to the 3' oxygen of its neighbor's pentose ring in one direction via a phosphodiester linkage. This end is referred to as the "5' end" if its phosphate is not linked to the 3' oxygen of a neighboring nucleotide. Further, if its 3' oxygen is not linked to the 5' phosphate of a neighboring nucleotide, then this end is of referred to as the 3' end. As used in the context of instant invention, every oligonucleotide is said to have 5' and 3' ends.

The term "probe" refers to an oligonucleotide (synthetic or occurring naturally), that is significantly complementary to a "fragment" and forms a duplexed structure with at least one strand of the replicated "nucleic acid analyte". It may be used to immobilize the replicated "analyte" strand or report the presence of the "nucleic acid analyte".

The term "complementary strand" refers to a nucleic acid sequence strand which, when aligned with the nucleic acid sequence of one strand of the target nucleic acid such that the 5' end of the sequence is paired with the 3' end of the other sequence, is in antiparallel association forming a stable "duplexed" structure. Complementarity need not be perfect. Stable duplexes may be formed with mismatched bases or when a label moiety has been placed in the phosphodiester-deoxyribose backbone instead of a nucleotide, causing the absence of a nucleotide base pairing at that point in the duplex.

The term "detection probe" refers to a nucleic acid "probe" having a replication inhibitor moiety at the 3' end that prevents the "probe"from being chain extended by a polymerase enzyme during nucleic acid replication. Further, the "probe" may be of such composition (e.g., peptide nucleic acid) as to render it inherently incapable of being chain extended by a polymerase enzyme. The "detection probe" will be of such a length and sequence as to be able hybridize to a portion of a nucleic acid analyte under appropriate conditions and annealing temperatures forming a duplex. The "detection probe" may incorporate one or more "labels". These labels may be coupled at any point in the probe and by any means.

The term "target domain" means a sequence complementary to a region of the target nucleic acid and positioned upstream of the replication inhibitor nucleotide. Target domains may be included in the detection probe and may include all or part of the probe binding domain.

The term "probe/analyte hybrid" or "hybrid" refers to a duplex formed between the detection probe and the nucleic acid analyte via base hybridization under the appropriate annealing temperature conditions.

The term "replication inhibitor moiety" refers to 3'-deoxynucleotides (e.g., cordycepin).

The term "non-participatory" will refer to the lack of participation of a probe or primer in a reaction for the amplification of a nucleic acid molecule. Specifically a non-participatory probe or primer is one will not serve as a substrate for, or be extended by, a DNA or RNA polymerase. A "non-participatory probe" is inherently incapable of being chain extended by a polymerase. It may or may not have replication inhibitor moiety.

The term "label" refers to any atom or molecule that can be used as a "reporter" or a "ligand", and which can be attached to a nucleic acid or protein. A label may be attached to an oligonucleotide during chemical synthesis, coupled though a chemically reactive group or incorporated on a labeled nucleotide during nucleic acid replication. Some of these labels may be ligands and serve as members of a binding pair. Such ligands are incorporated into the probe in such a manner as to enable the ligand to react, if necessary, with a second member of a binding pair. Additionally, labels may be reporter molecules and may also be incorporated into the probe in a manner similar to ligand label. Such reporter molecules may be chromogenic, radioactive, chemiluminescent, bioluminescent or fluorescent.

The term "reporter" refers to a "label" that can be used to provide a detectable (preferably quantifiable) signal. Reponers may provide signals detectable by fluorescence, luminescence, radioactivity, colorimetry, X-ray diffraction or absorption, magnetism, enzymatic activity, and the like.

The term "reporter pair" refers to matched fluorphores or enzymes capable of generating a detectable signal by virtue of their relative proximity to each other.

The term "ligand" or "reactive ligand" will refer to a "label" that can act as one member of a binding pair, which include but is not limited to antibodies, lectins, receptors, binding proteins, peptides or chemical agents.

The term "binding pair" includes any of the class of immune-type binding pairs, such as antigen/antibody or hapten/anti-hapten systems; and also any of the class of nonirnmune-type binding pairs, such as biotin/avidin; biotin/streptavidin; folic acid/folate binding protein; complementary nucleic acid segments, including peptide nucleic acid sequences; protein A or G/immunoglobulins; and binding pairs, which form covalent bonds, such as sulfhydryl reactive groups including maleimides and haloacetyl derivatives, and amine reactive groups such as isotriocyanates, succinimidyl esters and sulfonyl halides.

The term "capture reagent" refers to any reagent immobilized on a support that is capable of reacting with or binding a ligand incorporated either in the "detection probe" or the "nucleic acid analyte". Capture reagents are typically members of immunoreactive or affinity reactive members of binding pairs.

The term "reporter reagent'' refers to a "reporter" coupled to one member of a binding pair. Typically the member of the binding pair is an antibody or some immuno-reactive or affinity-reactive substance.

The term "primer" refers to an oligonucleotide (synthetic or occurring naturally), which is capable of acting as a point of initiation of nucleic acid synthesis or replication along a complementary strand when placed under conditions in which synthesis of a complementary stand is catalyzed by a polymerase. Wherein the primer contains a sequence complementary to a region in one strand of a target nucleic acid sequence and primes the synthesis of a complementary strand, and a second primer contains a sequence complementary to a region in a second strand of the target nucleic acid and primes the synthesis of complementary strand; wherein each primer is selected to hybridize to its complementary sequence, 5' to any detection probe that will anneal to the same strand.

The term "primer directed nucleic acid amplification" or "prirner-directed amplification" refers to any method known in the art wherein primers are used to sponsor replication of nucleic acid sequences in the linear or logarithmic amplification of nucleic acid molecules. Applicants contemplate that primer-directed amplification may be accomplished by any of several schemes known in this art, including but not limited to the polymerase chain reaction (PCR), ligase chain reaction (LCR) or strand-displacement amplification (SDA).

The term "nucleic acid replication composition" refers to a composition comprising the ingredients necessary for performing, nucleic acid replication including nucleotide triphosphates, divalent ions, reaction buffer, and in the case of primer-directed replication at least one primer with appropriate sequences, DNA or RNA polymerase and other necessary proteins.

The term "detection" will refer to the ability to identify a nucleic acid fragment of interest by the instant method.

The term "lateral flow format" or "lateral flow assay" will refer to a method of detecting a nucleic acid analyte/probe hybrid, where a sample containing the hybrid is placed on a test strip consisting of a bibulous material, and the sample is wicked along the surface of the test strip by capillary action, coincidentally reacting with various reagents in the strip. The hybrid is immobilized at a point on the strip via the interaction of a reactive ligand incorporated within the probe and a reactive member of a binding pair on the strip. The immobilized hybrid is then detected using reporters.

The stability of a nucleic acid duplex is measured by the "melting temperature" or "Tm", which under specified conditions, is the temperature at which half of the base pairs have been dissociated.

The term "probe binding domain" refers to a portion of a probe complementary to a second probe and can be complementary to the target nucleic acid. Within the context of the present invention probe biding domains are typically found on signal generating and signal modifying detection probes and serve to bind these probes together in the homogenous detection probe system.

The term "signal generating detection probe" or "SGDP" means a probe comprising a target domain, one member of a reporter pair, a probe binding domain and will be inhibited from extending at the 3' end during primer directed amplification. The method of inhibiting extension may rely on the presence of a nucleic acid tether, molecular spacer or a replication inhibitor moiety.

The term "signal modifying detection probe" or "SMDP" means a probe comprising, one member of a reporter pair and a probe binding domain and will be inhibited from extending at the 3' end during primer directed amplification. The method of inhibiting extension may rely on the presence of a nucleic acid tether, molecular spacer or a replication inhibitor moiety.

The term "homogenous detection probe system" or "HDPS" refers to a system at least two probes wherein at least one probe comprises a first member of a reporter pair, a target domain, a first probe binding domain and a replication inhibitor moiety and the second probe comprises a second member of a reporter pair and a second probe binding domain complementary to the first probe binding domain wherein the first and second reporter ligands are capable of reacting with each other to produce a detectable signal. Within the system the individual probes may be chemically tethered or linked through a nucleic acid segment.

The term "bivalent detection probe" or "BVDP" refers to a modified detection probe comprising two oligonucleotides linked by a molecular spacer. In one embodiment, one oligonucleotide is designed to participate as a "detection probe", whereas, the second oligonucleotide is designed to act as "capture arm sequence", which may serve to immobilize the modified probe by hybridizing with other nucleic acid fragments bound to a suitable support. It may also serve as a sequence that will hybridize to an oligonucleotide that will act as a probe or "reporter sequence" to detect the "analyte /BVDP" hybrid. In the second embodiment, the BVDP may act as an element in the homogeneous detection probe system.

The term, "molecular spacer" or "chemical tether" refers to heterobifunctional cross-linking agents, phosphodiester bridges that can change the polarity of the phosphodiester backbone using a 5',5' or 3',3'-phosphodiester bridge, carbon or carbon-oxygen spacer arms.

The present invention provides a method for the sensitive and efficient detection of specific nucleic acid analytes, important in the field of medical veterinary, agricultural, food and environmental diagnostics. Nucleic acid analytes may be derived from human, animal, or microbiological sources or habitats including body fluids, microbial culture fluids, crop materials, soils and ground waters.

The present invention provides a method for the detection of specific nucleic acid analytes involving a unique detection probe. The probe is 3' blocked or non-participatory and will not be extended by, or participate in, a nucleic acid amplification reaction. Further, the probe is designed to contain sequence that is complementary to some part of the nucleic acid analyte to be detected. The complementary sequence allows for the hybridization capture of the analyte by the probe. Additionally, the probe incorporates a label that can serve as a reactive ligand that acts as a point of attachment for the immobilization of the probe/analyte hybrid or as a reporter to produce detectable signal.

### DESIGN OF THE DETECTION PROBE (DP)

There is described a unique non-participatory detection probe useful for the capture, immobilization and detection of an amplified nucleic acid analyte (not part of the claimed invention). The probe may be several hundred bases in length where 25-65 base is preferred. The instant probe is versatile and may be designed in several alternate forms.

The 3' end of the probe is blocked from participating in a primer-extension reaction by the attachment of a replication inhibiting moiety. Typical replication inhibitors moieties will include but are not limited to, dideoxynuleotides, 3-deoxynucleotide, a sequence of mismatched nucleosides or nucleotides, 3' phosphate groups and chemical agents. Within the context of the present invention cordycepin (3' deoxyadenosine) is preferred.

In a preferred embodiment of the present invention, the replication inhibitor is covalently attached to the 3' hydroxy group of the 3' terminal nucleotide of the non-participatory detection probe during chemical synthesis, using standard cyanoethyl phosphoramidite chemistry. This process uses solid phase synthesis chemistry in which the 3' end is covalently attached to an insoluble support (controlled pore glass -CPG) while the newly synthesized chain grows on the 5' terminus. Within the context of the present invention, 3-deoxyribonucleotides are the preferred replication inhibitors. Cordycepin, 3-deoxyadenosine, is most preferred. Since the cordycepin will be attached to the 3' terminal end of the detection probe, the synthesis is initiated from a cordycepin covalently attached to CPG. 5-dimethoxytrityl-N-benzoyl-3-deoxyadenosine (cordycepin). 2-succinoyl-long chain alkylamino-CPG (Glen Research, Sterling, VA). The dimethoxytrityl group is removed and the initiation of the chain synthesis starts at the deprotected 5' hydroxyl group of the solid phase cordycepin. After the synthesis is complete, the oligonucleotide probe is cleaved off the solid support leaving a free 2' hydroxyl group on the 3'-terminally attached cordycepin. Other reagents can also be attached to the 3' terminus during the synthesis of the non-participatory detection probe to serve as replication inhibitors. These include, but are not limited to, other 3-deoxyribonucleotides, biotin, dinitrophenol, fluorescein, and digoxigenin, which are also derivatized on CPG supports (Glen Research, Sterling, VA, Clonetech Laboratories, Palo Alto, CA).

It is understood that the detection probe may be RNA or DNA or a synthetic nucleic acid, however, it will contain some sequence sufficiently complementary to the nucleic acid analyte to be detected that will permit hybridization between the detection probe and the analyte.

### Homogeneous Detection Probe System (HDPS)

The detection probe can be modified to enable homogeneous detection without the required immobilization of the analyte/probe hybrid. The HDPS refers to a system of two probes wherein one probe, the signal generating detection probe (SGDP), contains a target domain, a probe binding domain, and one member of a reporter pair. The second probe, signal modifying detection probe (SMDP); contains a probe binding domain complementary to the first probe binding domain, and a second member of the reporter pair. When the probes hybridize to each other, the regib sensitive members of the reporter pair are brought into close proximity to facilitate energy transfer. In this way, when the two probes are in a duplex configuration, signal modification due to energy transfer occurs. In one embodiment, the complementary SGDP and SMDP can reside-on two separate nucleic acid strands.

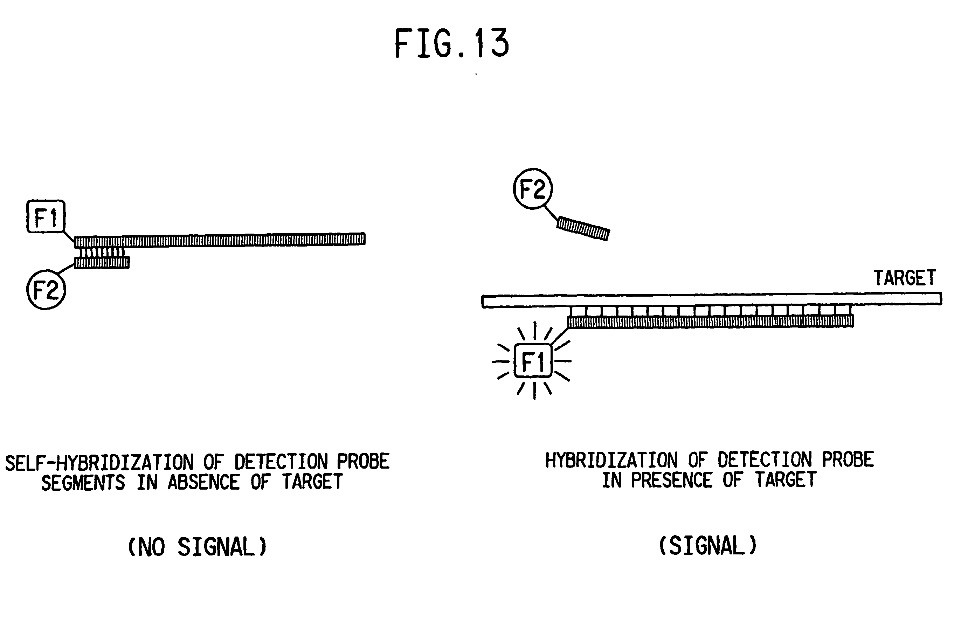

As depicted in Figure 13, if no target is present, the two probes hybridize together. Consequently, in the hybridized probe duplex, energy transfer will occur between the reporters resulting in an altered signal. As the target nucleic acid accumulates during PCR, the larger SGDP containing the target domain will hybridize to the target nucleic acid, displacing the smaller SMDP. The members of the reporter pair will thus be separated spatially resulting in signal modification. The resultant signal can then be detected without modulation or alteration of the signal generation potential. By selection of the appropriate fluorophores or signal generating reporter pairs, energy transfer can result in either absorption of fluorescence (quenching) or in a change in emitted light (wavelength shift). In either case, hybridization of the SGDP to its target nucleic acid prevents hybridization of the two probe strands and thus results in a detectable signal. The presence of a target nucleic acid sequence can therefore be detected directly in solution without the need for target immobilization.

The target domain of the probe would typically be 20 to 100 bases in length. However, this length depends on the required specificity for recognition of the target sequence and the differential thermal stability of the SGDP/SMDP duplex and the SGDP/target duplex. The Tm of the latter configuration should exceed the Tm of the SGDP/SMDP duplex by at least 10°C.

In preferred embodiments, it is desired to position members of reporter pairs in the probe sequences so as to enable energy transfer between fluorophores, intra molecular quenching, or to enable enzyme channeling between proximally positioned coupled enzymes. For each embodiment, the spatial distance and types of fluorophores are important considerations.

Specifically, energy transfer between fluorophore reporter pair members can be achieved if the distance between them is within ca. 50A. Energy transfer can result in a shift in the wavelength of the emitted fluorescence or result in absorption (quenching) of the fluorescence emission (R. A. Cardullo et al., Proc. Natl. Acad. Sci. USA, 85, 8790 (1988)). A preferred distance between the two fluorophores is 5 to 12 bases within the helical region of the probe duplex assemblage created by hybridization. Typically, this distance can be achieved by positioning the reporter members at the 5' and 3' ends of the SGDP and SMDP probe strands, respectively. In contrast, when the SGDP probe is annealed to a target nucleic acid, the fluorophores are positioned at a distance greater than 50A. Energy transfer is therefore not possible. The resulting fluorescent energy emission could then be measured and would be indicative of the amount of target nucleic acid present.

The requirements for fluorophore reporter pairs which participate in energy transfer are well documented (L. E. Morrison, Anal. Biochem., 174, 101 (1988)). Generally, to achieve energy transfer, it is important to select the appropriate combination of fluorophores such that the emission spectrum of one fluorophore overlaps with the absorption or excitation spectrum of the other fluorophore. The following fluorophore combinations include commonly available suitable candidates for energy transfer:

| Fluorophore (F1) | Fluorophore (F2) |
|---|---|
| Pyrenebutyrate | b-Phycoerythrin |
| Fluorescein | Texas Red |
| Lucifer Yellow | Rhodamine |
| Lucifer Yellow | Texas Red |
| Fluorescein | Rhodamine |
| Fluorescamine | Fluorescein |
| I A EDANS | DABCYL |

One advantage of the homogeneous detection probe system is the reduced procedural and reagent complexities of detecting the formation of the SGDP/target complex. First, there is a direct and .proportional relationship between the formation of the SGDP/target hybrid and signal generation. Most importantly, the formation of the probe/target hybrid can be detected in the presence of the SGDP/SMDP complex, directly in solution. Since there is no need for a solid phase capture reagent or for a wash step to remove the excess SGDP/SMDP probe complex, real-time detection of product formation is possible. An additional advantage of the HDPS is that, depending on the type and amount of signal generated when the SGDP hybridizes to the target, amplification or replication of the target nucleic acid strand may not be necessary.

In addition, the HDPS is of further value in *in situ* detection of genes and mRNA expression within cells and microorganisms. Currently, background fluorescence arising from nonspecific adsorption and binding of probes constrains the detection of gene sequences and mRNA formation in cells. The homogeneous detection probe system diminishes background response since the signal is generated only when the probe is hybridized to the target sequence. When the SGDP and the SMDP are hybridized together, in absence of target, the signal is not generated or is readily differentiated due to a shift in signal wavelength. In flow cytometry and in *in situ* microscopy, the HDPS also provides a means of achieving increased signal to noise response.

### PRIMER DIRECTED AMPLIFICATION

The present method provides for the amplification of a target nucleic acid to produce a nucleic acid analyte. A variety of nucleic acid amplification methods are known in the art including thermocycling methods such as polymerase chain reaction (PCR) and ligase chain reaction (LCR) as well as isothermal methods and strand displacement amplification (SDA). Additional methods of RNA replication such as replicative RNA system (Qβ-replicase) and DNA dependent RNA-polymerase promoter systems (T7 RNA polymerase) are contemplated to be within the scope of the present invention.

Typically, in PCR-type amplification techniques, the primers have different sequences and are not complementary to each other. Depending on the desired test conditions, the sequences of the primers should be designed to provide for both efficient and faithful replication of the target nucleic acid. Methods of PCR primer design are common and well known in the art. (Thein and Wallace, "The use of oligonucleotide as specific hybridization probes in the Diagnosis of Genetic Disorders", in *Human Genetic Diseases: A Practical Approach,* K. E. Davis Ed., (1986) pp. 33-50 IRL Press, Herndon, Virginia); Rychlik, W. (1993) In White, B. A. (ed.), Methods in Molecular Biology, Vol. 15, pages 31-39, PCR Protocols: Current Methods and Applications. Humania Press, Inc., Totowa, NJ.)

When the ligase chain reaction (LCR) is used for replication of a target double stranded nucleic acid, two sets of target-specific primers will be required. The members of one set of primers are complementary to adjacent sequences found on a given strand of the target, while the members of the second set are complementary to adjacent sequences on the opposite strand. In this way, a set of adjacent primers is specific for each target strand. During the replication process, the target nucleic acid is heated to denature the two target strands. The four complementary oligonucleotide primers comprising the two primer sets are then hybridized near their melting temperature to the separated target strands. A thermal-stable ligase will covalently attach the adjacent primers on each target strand. Only adjacent primers that are perfectly complementary to the target will be ligated together. In this way, the products from the first stage of ligation become targets for the next round of ligation. The products thus increase exponentially with continued cycles of target denaturation, primer hybridization and ligation steps.

The requirements for non-complementarity between primers, size, base composition and melt temperature requirements of the primers tend to be similar to those stated above for PCR replication. Generally, primers for LCR replication should be sufficiently long so that each will preferentially bind to its specific binding site on the target nucleic acid. To insure specificity of ligation, reactions can be carried out near the melting temperature (Tm) of the oligonucleotide primers. At higher temperatures, distablization of the terminal bases at the junction between adjacent primers can form. This results not only in imperfect double helix but in a lower ligation rate.

Strand displacement amplification (SDA) offers an isothermal alternative to PCR for the amplification of nucleic acids and may be used to amplify either a single-stranded or double-stranded target. Materials necessary for SDA amplification include either one or two short primers containing an asymmetric restriction enzyme site, such as *Hincll*, an exonuclease-deficient DNA polymerase, *Hincll* restriction enzyme and the bases dGTP, DCTP, DTTP and deoxyadenosine 5'[α-thio]triphosphate (dATP[αS]).

If the target to be amplified is single-stranded, a single primer is used which binds to the target at its complementary 3' ends forming a duplex with a 5' overhang at each end. The 5' overhang strand of the primer contains a recognition sequence for the restriction enzyme, *Hincll.* An exonuclease-deficient DNA polymerase I extends the ends of the duplex using dGTP, dCTP, TTP and dATP[αS], which produces a hemiphosphorothioate recognition site. *Hincll* nicks the unprotected primer strand of the hemiphosphorothioate site leaving intact the modified complementary strand. The exo-polymerase extends the 3' end at the nick and displaces the downstream complement of the target strand. The polymerization/displacement step regenerates a nickable *HincII* recognition site: Nicking and polymerization/displacement steps cycle continuously producing a linear amplified single-stranded product of the target strand.

If a nucleic acid target is to be exponentially amplified, then two primers are used each having regions complementary to only one of the stands in the target. After heat denaturation, the single-stranded target fragments bind to the respective primers which are present in excess. Both primers contain asymmetric restriction enzyme recognition sequences located 5' to the target binding sequences. Each primer-target complex cylces through nicking and polymerization/displacement steps in the presence of a restriction enzyme, a DNA polymerase and the three dNTP's and one dNTP[αS] as discussed above. An in depth discussion of SDA methodology is given by Walker et al., *Proc. Natl. Acad*. *Sci. U.S.A.,* 89, 392, (1992).

Alternatively, asymmetric amplification can be used to generate the strand complementary to the detection probe. Asymmetric PCR conditions for producing single-stranded DNA would include similar conditions for PCR as described however, the primer concentrations are changed with 50 pmol of the excess primer and 1 pmol of the limiting primer. It is contemplated that this procedure would increase the sensitivity of the method. This improvement in sensitivity would occur by increasing the number of available single strands for binding with the detection probe.

### LABEL INCORPORATION INTO NUCLEIC ACIDS

It is an element of the present invention that the detection probe may contain various labels for the purposes of immobilization or signal generation. For the purposes of the present invention, labels of less than 2000 molecular weight are preferred where labels with molecular weights of less than 1000 are most preferred.

Positionally, labels can be incorporated either at the 5' or 3' ends of the probe. It is understood that any number of labels may be incorporated per probe, however, where the object is to achieve maximum sensitivity of the assay, a relatively large number of labels is preferred where a range of one to ten is most preferred.

The method of incorporation of the label into the nucleic acid sequences may be accomplished either by chemical or enzymatic means, or by direct incorporation of labeled bases into the target sequence. In a preferred approach, label incorporated sequences are prepared using labeled bases or primers during polymerase chain reaction. Labels incorporation can be accomplished either through the incorporation of primers modified with label(s) or using labeled dNTPs. Labeled primers can be prepared using standard oligonucleotide cyanoethyl phosphoramidite chemistry by substituting selected bases with labeled phosphoramidites or label-modified base phosphoramidites during chemical synthesis. Alternatively, if primers are prepared with modified bases containing a linkable molecular spacer, the label can be chemically linked to the spacer after chemical synthesis. Another method would make use of labeled dNTPs or amino-modified dNTPs which can be incorporated into a analyte nucleic acid sequence during the amplification procedure.

In a preferred embodiment of the present invention, the detection probe is labeled during chemical synthesis using standard cyanoethyl phosphoramidite chemistry. The labels were at attached at the 5' or 3' position or could be placed in any internal sequence position of the detection probe. Within the context of the present invention, labeled phosphoramidite reagents that possess 2-aminobutyl-1,3-propanediol backbone (Label ON™ Reagents. ClonTech, Palo Alto, CA) or 1-(1,2 diaminoethane)3-deoxyfructonic acid (Virtual Nucleotide™ Reagents, ClonTech, Palo Alto, CA) are preferred because they offer the advantage of multiple label incorporation for stronger reporter signal or multiple affinity binding sites for a probe/analyte hybrid capture. When the 2-aminobutyl-1,3-propanediol reagents are incorporated internally, the natural three-carbon internucleotide phosphodiester spacing is maintained so that duplex destabilization is minimized. The 1-(1,2 diaminoethane)3-deoxyfructonic acid reagents are also designed to be incorporated internally on the oligonucleotide. The 3-deoxyfructonic acid subunit is designed to mimic a 2-deoxyribose sugar moiety as a spacer unit that attaches the label to the oligonucleotide. In this wayn internucleotide distance is maintained and the duplex formation is stabilized.

### INCORPORATED REPORTER MOLECULES

Various reporter molecules may be incorporated into the sequence of the detection probe. Labeling of nucleic acids with radioactive or fluorescent molecules is well known in the art. For example, nucleic acids may be labeled on their 5' end using T4 polynucleotide kinase and ³²P gamma-labeled ATP. The T4 kinase specifically transfers the radiolabeled phosphate from the ATP to a 5'OH group of the nucleic acid. This method is particularly useful for the end-labeling of small DNA or RNA molecules. Alternatively, 3' end-labeling of DNA may be accomplished using a terminal deoxynucleotidyl transferase (TdT) and labeled 2-deoxynucleotides or their labeled analogs 3-deoxynucleotides and dideoxynucleotides. Labeled ribonucleotides may also be used with TdT. A variety of labels for DNA and RNA would include, but are not limited to, ³²P, ³⁵S, ¹²⁵I or ³³P, fluorescein, rhodamine, biotin and dinitrophenol. Methods for the labeling of nucleic acids are well known in the art and are described fully in Sambrook et al., *Molecular Cloning,* Cold Spring Harbor Laboratory Press., Vol 2, 9.34-9.37 (1989).

### METHODS OF ASSAY

The present invention provides a method for the hybridization, immobilization and detection of a replicated nucleic acid analyte.

### Supports

In some embodiments, it may be desirable for the detection probe or replicated analyte sequence to bind to a support for the immobilization of the analyte/probe hybrid. A variety of possible supports are contemplated. For example, suitable immobilization supports include, but are not limited to synthetic polymer supports, such as polystyrene, polypropylene, polyglycidylmethacrylate, substituted polystyrene (e.g., aminated or carboxylated polystyrene; polyacrylamides; polyamides; polyvinylchlorides, etc.); beads; agarose: or nitrocellulose, nylon etc. These materials may be used as films, microtiter plates, wells, beads, slides, particles, pins, pegs, test tubes, membranes or biosensor chips. Alternatively, the supports could comprise magnetic and non-magnetic particles. Methods for the attachment of binding molecules oh solid supports are well known to those skilled in the art and reviewed by H.Weetall, Immobilized Enzymes, Antigens, Antibodies and Peptides, (1975) Marcell Dekker, Inc., New York.

### Assay Reporters and Reagents

Suitable assay reporters will be designed to interact with the probe label and will be equipped with a signal producing system which will produce a detectable signal that can be quantified. Typical signal producing systems may include, but are not limited to radioactive labels, enzymes, or chemiluminescent or bioluminescent or fluorescent moieties. In order to effect detection of the analyte/probe hybrid, reporters will be incorporated into reporter reagents comprising a reporter molecule linked to an immuno-reactive or affinity reactive member of a binding pair. A typical reporter reagent will comprise an enzyme coupled to an antibody. Preparation of such reagents may be accomplished using methods well known to those skilled in the art (D. G. Williams, *J. Immun. Methods.* 79,261 (1984)).

Enzymes suitable for use in reporters include, but are not limited to, hydrolases, lyases, oxido-reductases, transferases, isomerases and ligases. Others are peroxidase, glucose oxidase, phosphatase, esterase and glycosidase. Specific examples include alkaline phosphatase, lipases, beta-galactosidase, horseradish peroxidase and porcine liver esterase. In embodiments where enzymes serve as reporters the substrate/enzyme reaction forms a product which results in a detectable signal, typically a change in color. In many cases, chromogenic substances are an additional requirement for the color reaction. Chromogenic reagents are chosen on the basis of the reporter enzyme used. Some typical enzyme/chromogen pairs included, but are not limited to; β-galactosidase with chloro-phenol red β-δ-galactopyranoside (CPRG), potassium ferrocyanide or potassium ferricyanide; horse-radish peroxidase with 3,3' diaminobenzidine (DAB); glucose oxidase with nitro-blue tetrazolium chloride (NBT), alkaline phosphotase with para-nitrophenyl phosphate (PNPP), or 5-bromo-4-chloro-3-indolylphosphate-4-toluidine (BCIP)/NBT. Methods for the use of chromatogenic substance with enzyme reactions are well known in the art and are fully described by Tijssen, P., Practice and Theory of Enzyme Immunoassays in Laboratory Techniques in *Biochemistry and Molecular Biology.,* eds., R. H. Burton and P. H. Van Knippenberg.. (1988).

Alternatively, reporter conjugates may make use of radioactive or fluorescent labels as the reporting moiety. Typical radioactive labels may include but are not limited to ¹²⁵I, ³⁵S, ³²P, and ³³P. Similarly, suitable fluorescent reporter molecules may include, but are not limited to fluorescein, rhodamine, rhodoamine₆₀₀, R-phycoerythrin, and Texas Red. Further, it is contemplated that reporter conjugates will incorporate chemiluminescent and bioluminescent labels such as enzyme-triggerable dioxetanes as exemplified by the alkaline phosphatase substrate Lumigen® PPD (Schaap, AP *Photochem Photobiol 1988* 47S:50S)

### Assay Format

A variety of possible assay formats are contemplated to be useful in the practice of the present invention. For example, many assays incorporating appropriate supports listed above can be envisioned including a high throughput microtiter plate format, a lateral flow format, direct detection from gels, later flow or microtiter format are used, *In Situ* PCR and *In Situ* immuno-PCR, where the lateral flow and microtiter formats are preferred.

Methods for lateral flow detection of immunoreactive and nucleic acid analytes are known in the art (see for example Weng et. al, (U.S. 4,740,468); Reinhartz et al., (WO 9307292); McMahon et al., (U.S. 5.310.650)). In the present invention, a nucleic acid fragment analyte is amplified using appropriate primers and primer-directed amplification processes in the presence of the replication inhibited detection probe to form an analyte/probe hybrid where both the analyte portion and the probe portion incorporate a different reactive label. A suitable label for incorporation into the detection probe is biotin and a suitable label for incorporation into the replicated analyte is digoxigenin.

Following amplification, a sample containing an analyte/probe hybrid is subjected to lateral flow detection. Preferred concentrations of hybrid range from about 100 ng/mL to about 100 ug per 1 ml sample. Typically the lateral flow assay device comprises a test strip made of a bibulous porous material, which is capable of allowing fluid samples to traverse across it in a lateral fashion by capillary action. At one end of the strip is the application zone, which receives a liquid sample containing amplified product and the analyte/probe hybrid. As mentioned, the hybrid contains two different reactive labels, one used to immobilize the hybrid, the other to report the presence of the hybride (analyte). Further, one label is one associated with the probe and the other is associated with the replicated analyte. One of the labels will be used to immobilize the hybrid, the other to report its presence. Further up the strip is the capture zone, which contains an immobilized capture reagent, irreversibly affixed to the strip. Capture reagents are generally members of binding pairs such as biotin/avidin or digoxigenin/anti-digoxigenin. The capture reagent is designed to bind to either the replicated analyte specific label or probe specific reactive label. When the test sample containing the hybrid has been applied to the application zone, the sample is moved down the test strip by capillary action. Upon reaching the capture zone, hybrid is immobilized by the interaction of the capture reagent and the reactive ligands in the hybrid. Continued movement of the sample fluid draws excess reagents and unbound hybrid past the capture zone. In the case where the reporter reagent comprises an enzyme, reaction buffer containing the enzyme substrate, cofactors and chromogens are added to the test strip to effect detection. Interaction of the reporter enzyme with its substrate catalyses a reaction, which results in a detectable signal. Typically, a chromogenic substance, such as nitrobluetertazolium (NBT), is used in the reaction buffer for signal generation.

It will be appreciated that the lateral flow assay format is versatile and is not limited to the detection of a single analyte/probe hybrid. Rather, it is contemplated that a multiplicity of hybrids could be detected in a single assay by allowing for a variety of reactive labels, reporters and capture reagents.

### Microtiter Assay Format

In an alternate embodiment, the present method may be used in a high throughput microtiter plate assay format. Analyte/probe hybrids are prepared as described above. Microtiter plates of polystyrene or some suitable support material are coated with a member of a binding pair such as streptavidin. The binding pair is designed to react with a ligand that is incorporated into one portion of the analyte/probe hybrid. After washing and blocking the coated plates, samples containing the labeled hybrid are introduced into their respective well of the microtiter plate and incubated. If the probe portion of the hybrid is labeled with biotin, it serves to immobilize the hybrid to the surface of the well. After washing, a suitable reporter reagent is added to the well which is designed to bind to the analyte specific label. Reporters and reporter reagents, suitable for the lateral flow assay format, are also suitable for use in the microtiter plate format.

### Direct, in-gel detection of the product/probe hybrid:

The detection probe technology can also be applied to detection of a specific fragment that must be distinguished from a large background of nonspecific products. Typically in these cases, the nucleic acid products, after gel electrophoresis, are transferred to membranes and hybridized with a labeled, sequence specific probe. However, with the applicants method, the detection probe is already hybridized to the final product and the signal can be developed directly in the gel through the addition of the antibody conjugate and chromogenic substrate (Sun, et al., *BioTechniques*, 16. 782, (1994)). The specific fragment can therefore be identified rapidly without time-consuming membrane transfer and overnight hybridization procedures.

### Immuno-PCR:

Another example of an alternative application of the detection probe technology involves the immuno-PCR procedure. In this procedure, a non-nucleic acid analyte is detected by a specific antibody bearing a DNA label. This DNA label is then amplified with sequence specific primers by standard PCR technology. As measured by gel electrophoresis, the resultant product formed is indicative of the quantity of the analyte present (Sano et al., *Science*, 258, 120-122, (1992) and Hendrickson et al., *NAR,* 23, No. 3, 522-529, (1995)). As an alternative, the detection probe technology could be incorporated into the immuno-PCR procedure. The product/probe hybrid formed could then be detected in the lateral flow, microtiter plate assay or other formats.

In addition, antibodies directed towards different analytes could each contain a separate, analyte specific DNA label. Once the antibody binds to its respective analyte, the DNA labels could then be amplified in the presence of its sequence specific detection probe. Each probe would contain a different reactive ligand (e.g., biotin, digoxigenin, dinitrophenol, fluorescein), while the amplified DNA label products would have incorporated a single label (e.g., digoxigenin, fluorescein, DNP, biotin). The product/probe hybrids would then be captured by their respective membrane bound antibody and reported through a chromogenic or chemiluminescent substrate (see Multianalyte Detection Section).

### In situ PCR:

*In situ* PCR involves localized amplification of DNA or mRNA directly in the cells or tissues. The cells may be in suspension, in tissue sections or slices on glass slides, or adhered to tissue culture plates. An in-depth discussion of *in situ* PCR is given in Nuovo, et al., *PCR in situ Hybridization,* Raven Press, 214-306 (1994). Typically, a reactive ligand such as digoxigenin is incorporated into the PCR product and the product is then detected by an enzyme-labeled reporter antibody and substrate. Alternatively, the detection probe containing fluorescein, for example, could be added at the beginning of the PCR procedure. The resultant, localized PCR product/probe hybrid would cause the cells to fluoresce. The fluorescent positive cells could then be detected by a variety of methods, including fluorescent microscopy or flow cytometry, for example (Gibellini et al., *Analytical Biochemistry*, 228, 252-258, (1995)). With the present method, the incubation steps with the reporter antibody and substrate associated with traditional *in situ* PCR would, therefore, be eliminated. Another advantage of this method is the reduced background. Non-specific reaction product formed from the binding of the reporter antibody would be reduced.

### In Situ Immuno-PCR using detection probe technology:

A novel procedure in which the detection probe would offer further advantages include the use of the immuno-PCR conjugate for *in situ* detection of antigens. Procedures for tissue preparation and immunohistochemistry, are followed by fixation in 4% paraformaldhyde, embedding in paraffin and sectioning. After preparation, the slides with the tissue sections are stored at room temperature until processing at room temperature. Note, the slides are coated to prevent loss of the tissue sections during the protocol. The slides are deparafinized and rinsed in 100 %, 75% then 50% ethanol for two minutes each. Then, the slides are washed in phosphate buffered saline before being rinsed in the blocking serum for 20 minutes. After blocking, the excess serum is bloned off and the slides are incubated with the reporter antibody (DNA labeled antibody) overnight. Next, the PCR reaction is carried out in the presence of the detection probe, using the reaction mix directly in contact with the sections. The slides are subjected to 20 cycles of PCR, on the Hybaid Omnislide using the following conditions:

After amplification the slides are rinsed in 2 X SSC (for buffer solutions see Sambrook et al., Molecular Cloning: A Laboratory Manual - volumes 1,2,3 (Cold Spring Harbor Laboratory: Cold Spring Harbor, New York, 1989). The slides are then reacted with sireptavidin alkaline phosphatase conjugate. Chromogenic substrate is then added to the slides. This reaction can also be carried out on cells in culture, on glass slides, tissue culture plates or in suspension. Cells containing the antigen will be detected by a chromogenic precipitate.

The present invention is further defined in the following Examples. It should be understood that these Examples, while indicating preferred embodiments of the invention, are given by way of illustration only. Examples 1-7 are reference examples.

### EXAMPLES

### GENERAL METHODS

Suitable methods of genetic engineering employed herein are described in Sambrook et al., Molecular Cloning: A Laboratory Manual - volumes 1.2.3 (Cold Spring Harbor Laboratory: Cold Spring Harbor, New York, 1989), and in the instructions accompanying commercially available kits for genetic engineering. Unless otherwise specified all other standard reagents and solutions used in the following examples were supplied by J. T. Baker Co. (Phillipsburg, NJ).

Base sequences of various primers used throughout examples are listed below in Table 1.

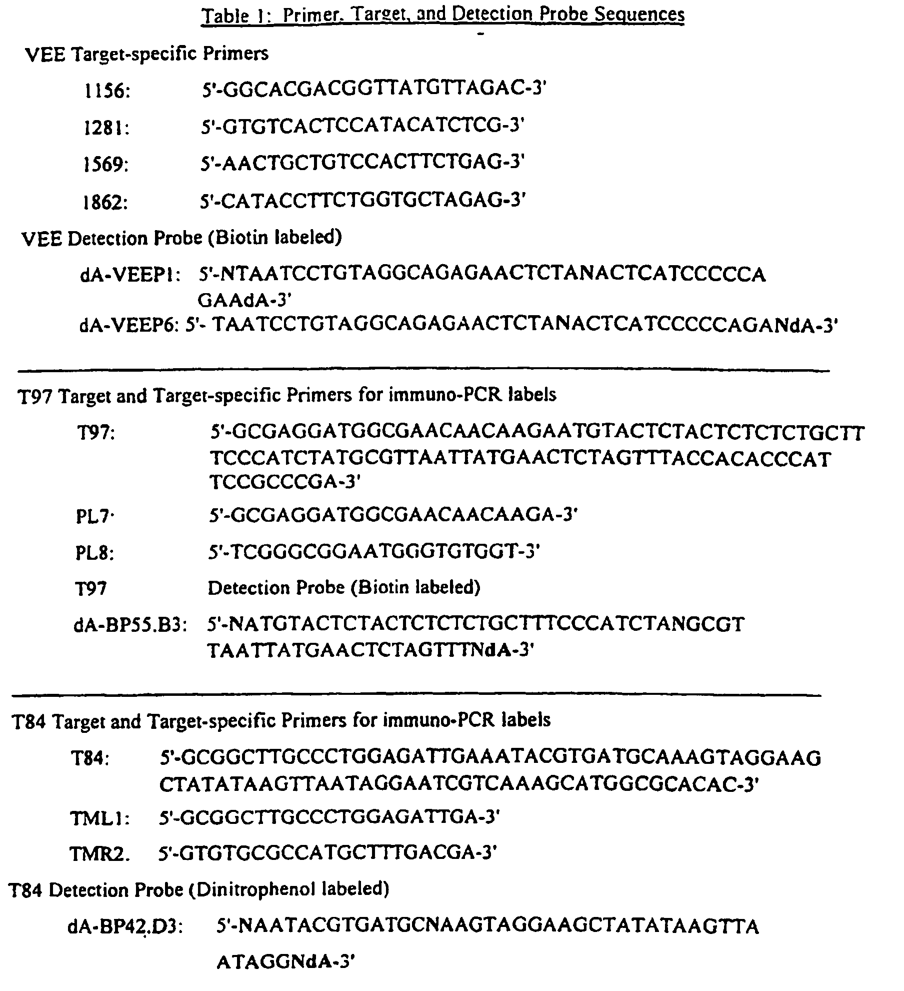

The labels (N) were substituted for nucleotides using labeled phosphoramidite reagents that possess 2 aminobutyl-1,3-propanediol backbone or 1-(1,2 diaminoethane) 3-deoxyfructonic acid. Cordycepin 5' triphosphate (3'deoxyadenosine) is indicated by, "dA".

### Nucleic Acid Amplification

Amplification of the target DNA in the presence of the biotinylated detection probe was performed as described. Various concentrations of the target DNA was amplified in the presence of 100 pmol each of primers, with 25 pmol of the detection probe and 200 uM dNTPs with a 10% substitution of dTTP with digoxigenin-11-dUTP (Boehringer Mannheim, Indianapolis, IN). Also included in the initial reaction mixture was 2.5 units of Taq polymerase (Perkin-Elmer Corp., Norwalk, CT) in a final volume of 100 ul PCR buffer (50 mM KCl, 10 mM Tris-Cl, pH 8.4. 1.5 mM MgCl₂, 0.01% gelatin). The DNA was amplified in a thermal cycler (Perkin-Elmer Corp.) as follows: denaturation at 94°C for 1 min, annealing at 50°C for 1 min, and extension at 72°C for 2 min for 35 cycles followed by an extension at 72°C for 8 min, denaturation at 94°C for 2 min, with a final annealing step at 50°C for 15 min.

### Synthesis of Detection probes for the Homogenous Detection Probe System (HDPS)

The two oligonucleotides probes, signal generating detection probe (SGDP) and signal quenching detection probe (SQDP), were chemically synthesized for the HDPS using standard cyanoethyl phosphoramidite chemistry. The SGDP, the signal-emitting detection probe of this assay system, is composed of two elements, a cordycepin at the 3' end and sulfhydryl group at the 5' end. The cordycepin is attached at the 3' end during the initiation step of the synthesis using a cordycepin coupled CPG column (Glen Research. Sterling. VA). The sulfhydryl is attached at the 5' end in the last step of chemical synthesis of the probe using C6 disulfide phosphoramidite (ClonTech, Palo Alto, CA) and standard cyanoethyl phosphoramidite chemistry. The SQDP, the signal quenching probe of the HDPS assay, is composed of two elements, a sequence complementary to the 5' end of the SGDP and 3' aliphatic primary amine group. The amine modifier is attached at the 3' end during the initiation step of the synthesis using a Fmoc aliphatic amine coupled CPG column (ClonTech, Palo Alto, CA). The Fmoc protection group is removed during standard oligonucleotide cleavage and deprotection.

The next step is to add the fluorophore-quenching pair, EDANS and DABCYL respectively, to the SGDP and the SMDP. This pair was selected because the absorbance spectrum of the DABCYL overlaps the emission spectrum of the EDANS (Excitation λₘₐₓ: 340 nm, Emission λₘₐₓ: 490 nm) which results in quenching of the EDANS signal through energy transfer. First, DABCYL is coupled to the 3' primary amine of SMDP using N-hydroxy-succinimide chemistry. In 200 µL of water, 200 µg of the oligonucleotide (5'TTA TGC CAT TN 3) in 100 mM sodium bicarbonate (pH 9.0) is reacted with 200 µL of succinimidyl ester of DABCYL (Molecular Probes, Eugene. OR), 10 mg/mL dissolved in dimethyl formamide (DMF). The DABCYL mixture is added in 50 µL aliquots every 2 hrs. The reaction is continually mixed and incubated for a period of 16 hrs. The reaction is stopped and precipitated by adding 40 µl of 3M sodium acetate (pH 5.6) and 800 µl of cold ethanol to remove the unreacted NHS-DABCYL. The second reaction is to couple EDANS to the 5' sulfhydryl group on the SGDP. The disulfide is cleaved by adding 20 µL of 500 mM dithioerythritol (DTT) to 200 ug of the SGDP oligonucleotide (5'S AAT GGC ATA ACA GGA TAA CAA TAA TCA AAT AAA AGT TTT AAA CAA ATA dA 3') in 200 µL water and the solution is allowed to stand at room temperature for 10 min. The DTT is extracted 4 x 800 µL ethyl acetate immediately prior to use. Next, 200 µl of 2 mM 1,5-iodoacetylated-EDANS (Molecular Probes, Eugene, OR) in 200 mM sodium bicarbonate (pH 9.0) is added to the thiol-modified oligonucleotide in the aqueous layer. The reaction is incubated at room temperature for 16 hrs. The unreacted iodoacetylated-EDANS is removed by using a Centricon™ 100 concentrator (Amicon, Inc., Beverly, MA) and a Sorvall® SM-24 rotor in a RC-5B centrifuge (Sorvall®, DuPont Co., Wilmington, DE), spun at 3500 rpm for 30 min at 20°C. 1.5 mL water are added to the retentate. The diluted retentate is again spun in a the Centricon™ 100 concentrator at 3500 rpm for 30 min at 20°C. This process is repeated three times. After the last step, the oligonucleotide is brought to a final volume of 200 µL.

Both the EDANS-labeled SGDP and DABCYL-labeled SMDP are purified on a 12% nondenaturing acrylamide gel (1.5 mm) in 1x tris borate EDTA buffer, pH 8.3(TBE). The electrophoresis is performed at 60 W constant power for 4 hrs. The unlabeled oligonucleotide is visualized by the shadow it casts when the acrylamide gel (in plastic wrap), lying on intensifying screen, is illuminated with a short wave UV lamp. The labeled oligonucleotide, located above the unlabeled oligomer is excised from the gel, placed in a 15 mL Falcon 2059 tube and crushed with a glass rod. One milliliter water is placed over the crushed gel and the oligonucleotide is eluted by agitating at room temperature overnight. The supernatant is removed and placed in an eppendorf tube. The concentration is determined by scanning the samples in a spectrophotometer and measuring the absorbance at 260 nm. Absorbance measurements are made at 336 nm and 490 nm for EDANS and DABCYL, respectively.

### EXAMPLE 1

### Amplification, Detection & Quantitation of the Amplified DNA-Replication Terminated Probe Product in a Lateral Flow Format

Example 1 demonstrates the use of the detection probe for the capture and detection of a nucleic acid fragment in the lateral-flow format.

### Amplificaition:

A segment of the E2 glycoprotein region of the Venezuelan equine encephalitis virus (VEE) genome (Kinney, et al., *Virol*., 170, 19,(1989) was amplified with different sets of primers. Primary amplification with primers, 1156 and 1862 (Table 1) resulted in a 707 bp product. A secondary amplification with nested primers. 1281 and 1569, was used to produce a 289 bp fragment. Amplification of the target DNA in the presence of the ligand labeled. 3' terminated detection probe was performed as described as follows.

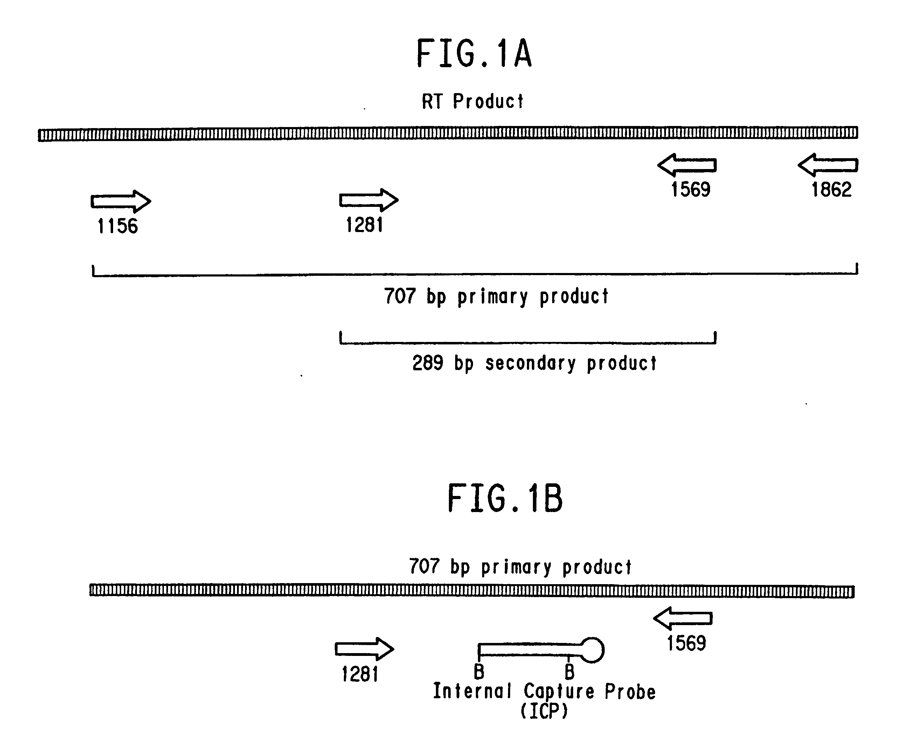

Various concentrations of the 707 bp primary amplification product were amplified in the presence of 100 pmol each of primers, 1281 and 1569/25 pmol of the detection probe/200 uM dNTPs with a 10% substitution of dTTP with digoxigenin-11-dUTP (Boehringer Mannheim. Indianapolis, IN)/2.5 units of Taq polymerase (Perkin-Elmer Corp.. Norwalk, CT) in a final volume of 100 ul PCR buffer (50 mM KCl/10 mM Tris-Cl, pH 8.4/ 1.5 mM MgCl₂/0.01% gelatin). The DNA was amplified in a thermal cycler (Perkin-Elmer Corp.) as follows: denaturation at 94°C for 1 min, annealing at 50°C for 1 min, and extension at 72°C for 2 min for 35 cycles followed by an extension at 72°C for 8 min, denaturation at 94°C for 2 min, with a final annealing step at 50°C for 15 min. Figure 1 illustrates the reverse transcription product and PCR products with location of primers and probe. Figure 1a, shows the reverse transcription (RT) product and the relative positions of the outer primers. 1156 and 1862. and the nested primers. 1281 and 1569. The primary amplification product was a 707 bp fragment while the secondary product was a 289 bp fragment. Figure 1b shows the 707 bp target fragment and relative position of the nested primers and the biotinylated detection probe (DP), terminated at the 3' end so that it will not participate in the amplification.

### Membrane Preparation

Streptavidin (2 mg/mL; Zymed Laboratories, Inc., San Francisco, CA) is printed onto 15 cm x 15 cm nitrocellulose sheets (AE98; Schleicher and Schuell, Keene, NH) using an ink jet printer. The sheets are then cut into identical strips, 5 mm wide by 5 cm long. Approximately 340 ng of streptavidin are deposited in a 1 mm by 5 mm line (capture zone). 2 cm from the bottom of the strip. The membranes are stored desiccated at room temperature for up to one year.

One microliter of the digoxigenin labeled PCR product, annealed to its biotinylated detection probe, is mixed with 19 uL of Lateral Flow Buffer (10 mM Tris pH 8.0. 150 mM NaCl, 1 mM MgCl₂, 0.1 mM ZnCl₂, 0.5% BSA, 0.1% Triton X-100) in a well of a 96-well, flat bottom, polystyrene microtiter plate (Dynatech Laboratories, Inc., Chantilly, VA). The tip of the strip containing the streptavidin capture zone is placed into the well for 5 min allowing the labeled PCR target to be pulled, by capillary action, up the strip and across the capture zone. The membrane is then transferred manually using forceps through a series of semimicro cuvettes containing the appropriate buffers in which the capture zone is completely submerged: The strip is first blocked in 30% goat serum (Sigma Chemical Co., St. Louis, MO), diluted in Buffer A (100 mM Tris-Cl, 150 mM NaCl, pH 9.5), for 5 min followed by an 8-12 min incubation in a 1:500 dilution of an anti-digoxigenin alkaline phosphatase conjugate (Boehringer Mannheim) in 3% goat serum. The strip is then washed in Buffer A for 3 min, Buffer B (100 mM Tris, 100 mM NaCl, 50 mM MgCl₂, pH 9.5) for 2 min, and then soaked in the NBT/BCIP (nitro blue tetrazolium/5-bromo-4-chloro-3-indolyl phosphate) substrate solution (Moss, Inc., Pasadena, MD) until color develops (3 min). After development, the strip is rinsed in distilled water and dried. The total time of the assay is 26-30 minutes.

### Quantitation

Aliquots of the PCR products were run alongside DNA quantitation standards (GenSura Laboratories, Inc., Del Mar, CA) on 2% agarose gels containing 0.5 ug/ml ethidium bromide. The PCR products were quantified against the standards using the Eagle Eye II Video System (Stratagene, La Jolla, CA) to record the image and the NIH Image Analysis 1.55 program (written by Wayne Rasband at NIH and available from the Internet by anonymous ftp from zippy.nimh.nih.gov) to quantify the image.

The lateral flow detection was quantified by measuring the mean pixel density of the capture zones minus the background signal on the membranes using the Eagle Eye II System and the NIH Image Analysis program as described above.

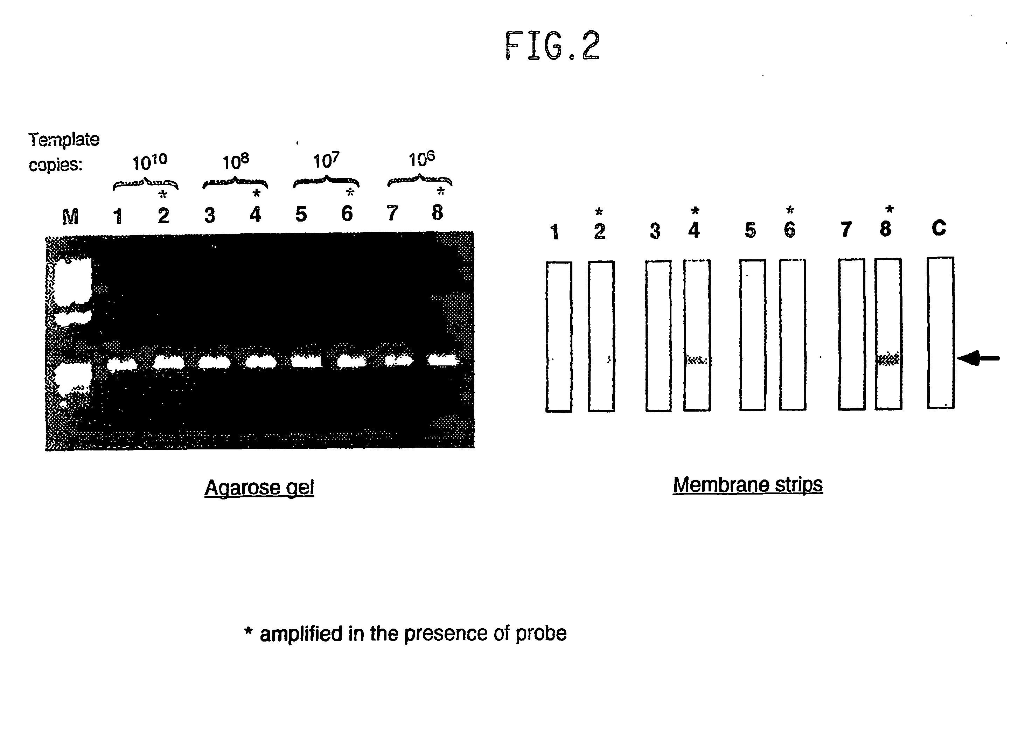

The results of the lateral flow detection are seen in Figure 2 which shows the agarose gel and lateral flow detection of the nested 289 bp amplified product with and without the biotinylated detection probe. Samples 1-8 are the result of the 707 bp primary amplification product amplified in the presence or absence of 5 pmol of the dA-VEEP1 probe. Samples 1 and 2 contained 2 x 10¹⁰ copies, lanes 3 and 4 contained 2 x 10⁸ copies, samples 5 and 6 contained 2 x 10⁷ copies, and samples 7 and 8 contained 2 x 10⁶ copies. One microliter of the products was detected on a 2%, ethidium bromide-stained gel as seen in Panel A and on the modified lateral flow membranes of Panel B. Control strip sample, C, contained no DNA. Even-numbered samples contained the probe whereas odd-numbered samples contained no probe. The streptavidin capture zone on the membranes is indicated by the arrow.

### EXAMPLE 2

### Detection of the Amplified Viral DNA-Replication Terminated Detection Probe Product in Microtiter Plate Assays

Example 2 demonstrates capture and detection of amplifed viral DNA using the detection probe in both the lateral flow and microtiter plate assay format.

### Plate Preparation:

The wells of a 96-well, flat bottom, polystyrene microtiter plate (MaxiSorp, Nunc, Inc., Naperville, IL) were coated with 100 ul of 10 ug/ml streptavidin for 1 hour at room temperature and then washed three times in TBS/Tween (25 mM Tris, pH 7.4, 50 mM NaCl. 0.05% Tween-20) with an automatic plate washer. The wells were then treated with 200 ul of Blocking Buffer (10 mM sodium phosphate, pH 7.4, 150 mM NaCl, 2% BSA, 10% β-lactose, 0.02% sodium azide) for 1 hour and washed again, three times.

Amplified analyte/probe hybrid samples were prepared as described in Example 1 and were diluted 1:100 in TBS/Tween, introduced into the wells, and incubated for 1 hr at room temperature, followed by another three washes in TBS/Tween. A 1:1000 dilution of the anti-digoxigenin alkaline phosphatase conjugate was added to the samples for 1 hr at room temperature and the wells were, again, washed three times. The signal was then developed in the presence of the chromogenic substrate, *p*-NPP (*p*-nitrophenylphosphate: Kirkegaard and Perry Laboratories. Inc., Gaithersburg, MD), or the chemiluminescent substrate. CSPD (Disodium 3-(4-methoxyspiro{1,2-dioxetane-3,2'-(5'-chloro)tricyclo [3.3.1.1^{3,7}] decan}-4-yl)phenyl phosphate) (Tropix, Bedford, MA).

### Quantitation of Detection

For the chromogenic microtiter plate assay using the *p*-NPP substrate, the optical density of the precipitate was measured at 405 nm by a Thermomax Microplate Reader (Molecular Devices Corp., Menlo Park, CA). An ML3000 Microtiter Plate Luminometer (Dynatech Laboratories. Inc.) was used to measure the relative light units emitted by the enzyme induced decomposition of the CSPD substrate for the chemiluminescent assay.

### EXAMPLE 3

### Demonstration of Lack of Interference of Replication 3' Terminated Probe on Product Yield. Determination of Optimal Probe Concentration and Comparison of Detection Methods

Example 3 shows that the detection probe does not interfere with the amplified product yield and teaches how the probe concentration may be optimized for a particular application. Included are comparisons of the detection methods.

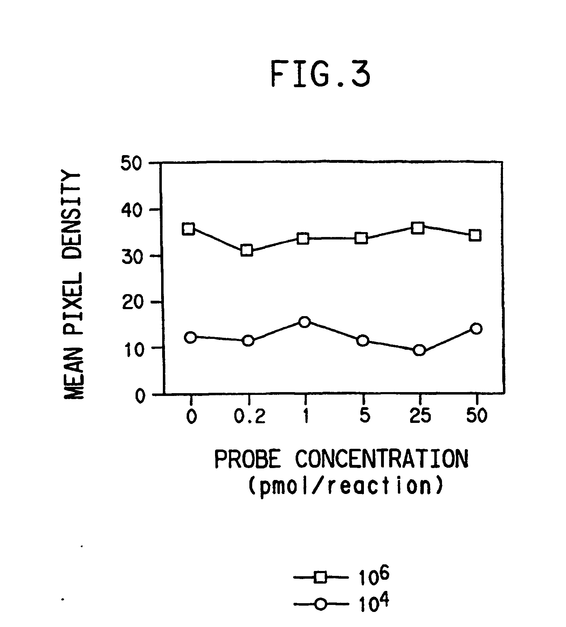

PCR reactions were performed using the primers and targets as described in Example 1 across a range of template and probe concentrations. Two initial target concentrations, 10⁶ copies and 10⁴ copies, of the 707 bp VEE primary PCR product were amplified in the presence of varying probe concentrations of 0, 0.2, 1, 5, 25, 50 pmol/reaction. Aliquots of the products were electrophoresed on a 2% agarose gel stained with 0.5 ug/ml ethidium bromide and were quantified using the Eagle Eye II Video System (Stratagene, La Jolla, CA) and the NIH Image Analysis 1.55 program. The results are plotted in Figure 3 which shows the effect of probe concentration on amplified product yield.

As can be seen in Figure 3 no effect on product yield was observed with the increased probe concentrations as determined by quantitation of the band intensity on the agarose gel.

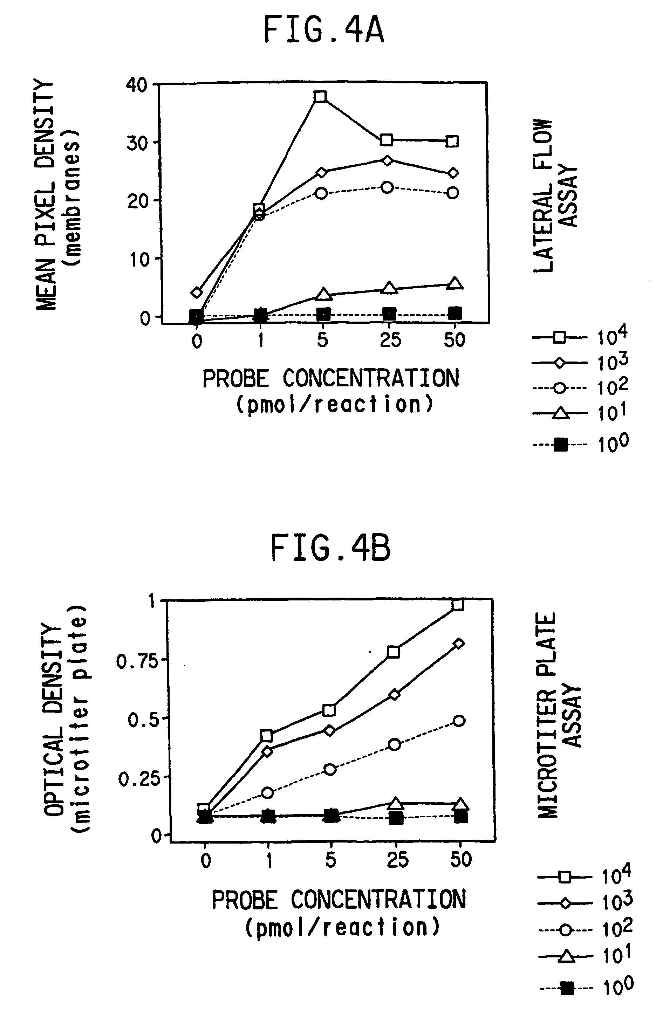

Figure 4 analyzes the effect of probe concentration and template copy number on product yield. Panel A represents detection of the analyte/probe hybrid in the lateral flow format and quantification of the intensities of the streptavidin capture zones. Values are expressed as mean pixel densities of the capture zone signal minus the strip background. Panel B reflects the detection of the products in the microtiter plate assay.

As seen in Figure 4, when one to ten thousand copies of template were amplified in the presence of increasing probe concentrations, as few as 10 copies (10¹) of initial target DNA could be detected in the lateral flow (Panel A) and microtiter plate (Panel B) formats. The target could be detected with as little as 5 pmol probe, although the optimal amount of probe across all template concentrations tested was 25 pmol.

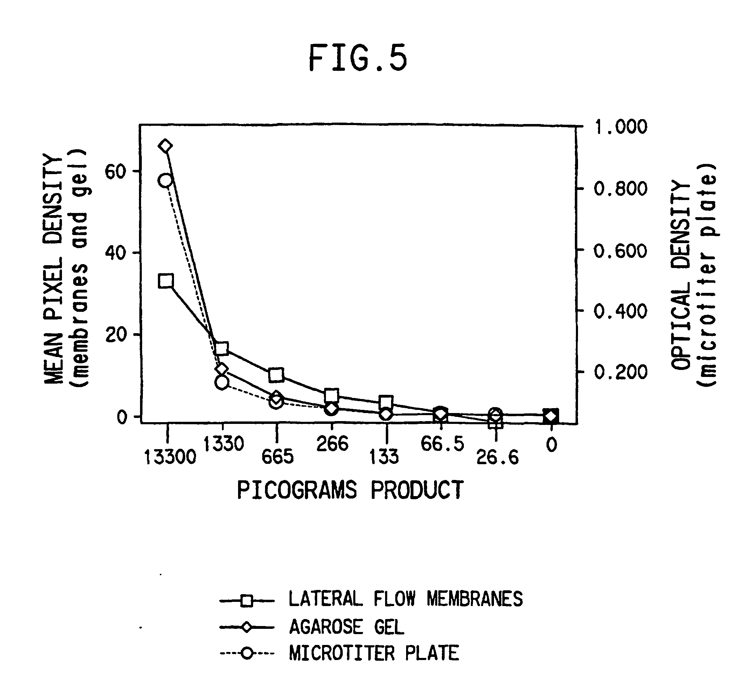

Similarly, the detection of the 289 bp amplified products by ethidium bromide-stained agarose gel electrophoresis, lateral flow, and microtiter plate assays was also compared. Serial dilutions of a PCR product that was amplified in the presence of 25 pmol of the detection probe were detected by agarose gel electrophoresis, lateral flow, or the microtiter plate assay as described above. Figure 5 illustrates the comparison of the detection of these amplified products by gel electrophoresis, lateral flow, and microtiter plate assays as measured and quantified by the Eagle Eye II Video System (Stratagene, La Jolla. CA) and the NIH Image Analysis 1.55 program. Mean pixel density of the gel bands and membrane capture zones are illustrated on the left axis while the optical density measurements (405 nm) from the microtiter plate assay are on the right.

As seen in Figure 5 the detection limit for the lateral flow assay was approximately 100 pg (520 amol) of PCR product while the limits for the traditional gel electrophoresis and microtiter plate assay were both approximately 500 pg (2.6 fmol).

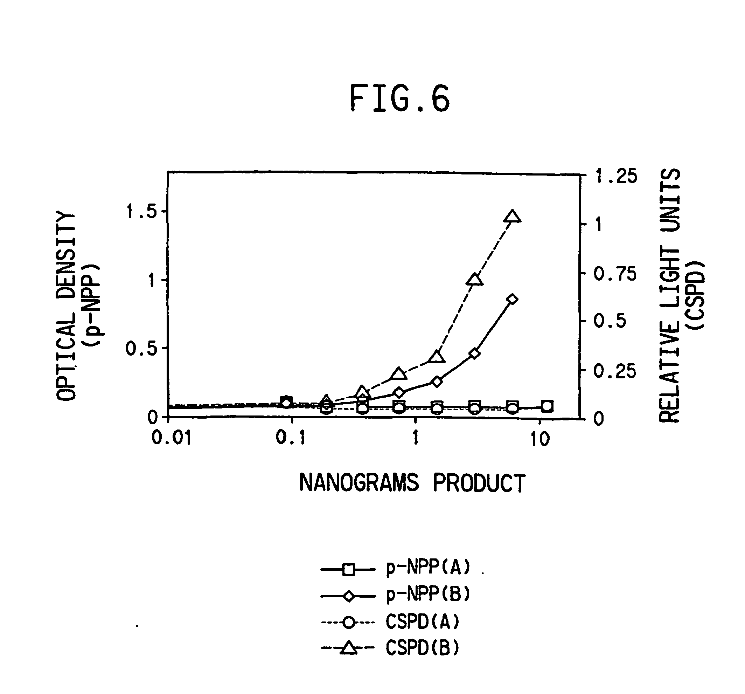

Lastly the high throughput microtiter plate assays using either chromogenic or chemiluminescent substrates for alkaline phosphatase were also compared and are illustrated in Figure 6. In Figure 6 diamond and square data points represent the optical density (405 nm) of samples, A (minus probe) and B (plus probe), developed in the presence of the *p*-NPP substrate and measured and quantitated on the Thermomax Microplate Reader (Molecular Devices Corp.). The circle and triangle data points reflect the relative light units of the samples developed in the presence of the chemiluminescent substrate. CSPD, and measured by an ML3000 Microtiter Plate Luminometer (Dynatech Laboratories, Inc.). Data points represent serial dilutions of the products. Optical density was illustrated on the left abscissa and the relative light units are on the right.

As seen in Figure 6 the detection sensitivity with the chemiluminescent substrate was somewhat higher than the sensitivity resulting from the chromogenic substrate as determined by measuring the slope of the lines. The use of the chemiluminescent substrate for the microtiter plate assay increased sensitivity close to that of the lateral flow method (about 200 pg).

### EXAMPLE 4

### Determination of Fate of 3' Terminated Detection Probe

It has been suggested in the literature that hybridization probes present during target amplification are subject to degradation (Longley et al., *(Nuc. Acids. Res*., 18, 7317, (1990); Lewis et al., *Nuc. Acids. Res*., 22, 2859, (1994)). Example 5 demonstrates that the detection probe is sufficiently resistant to degradation under the conditions of the present method. The probe/target interaction was studied to ascertain the fate of the probe after 35 cycles of amplification.

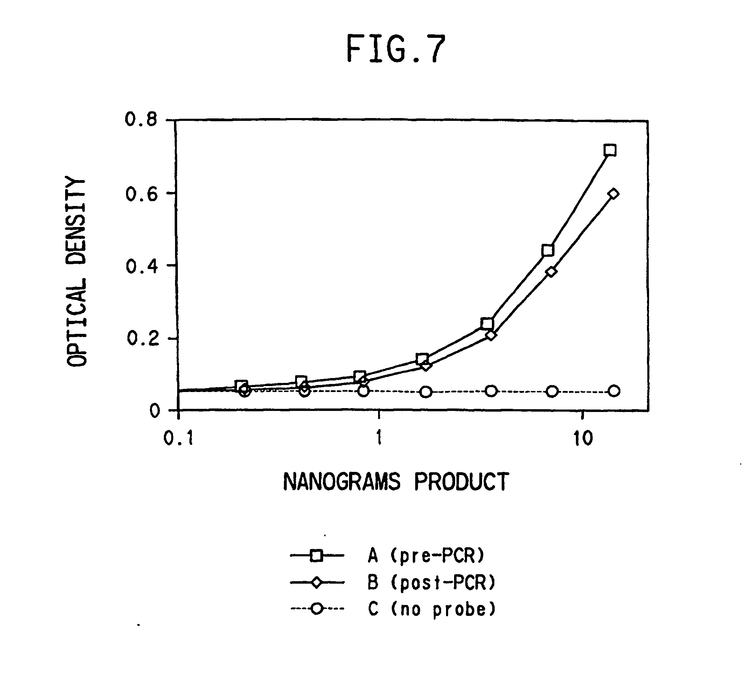

The 707 bp VEE template was amplified as previously described in the presence or absence of 5 pmol of the biotinylated dA-VEEP1 probe (Table 1). A microtiter plate assay was performed on the "plus probe'' sample, the "minus probe" sample, and the "minus probe" sample that had probe added after the PCR cycling was complete. This post-PCR captured product was denatured at 94°C for 2 min, incubated at 50°C for 15 min to allow the probe to anneal, and then cooled on ice. Results are shown in Figure 7. Figure 7 sample A corresponds to the "plus probe'' (13.3 ng/uL); sample B (14.3 ng/uL) corresponds to the "minus probe" sample that had probe added after the PCR cycling was complete and sample C corresponding to the "minus probe". Data points represent serial dilutions of the products.

The data in Figure 7 suggests that the probe, when added at the beginning of the PCR reaction, was not adversely affected by the multiple amplification cycles in that the detection of the product was relatively the same as when the probe was added at the end of the PCR reaction.

In addition, the specific placement of the biotin at the 5' end of the probe was also investigated to test whether the 5' modification acts to block the 5'-3' exonuclease activity of the *Taq* DNA polymerase. A new probe, dA-VEEP6, was designed with the biotins moved from the 5' end (Table 1). Amplification was performed as previously described in the presence of low concentrations (0.2 and 1.0 pmol per reaction) of the probes--concentrations within the linear range of vertical flow detection. The yield of products generated in the presence of the 5' unmodified probes and the detection of those products in the lateral flow format were similar to those amplified in the presence of the dA-VEEP1 probe (Table 2). This suggests that the lack of the 5' modification did not cause an increase in probe degradation as would be evidenced by signal loss.

**Table 2**

| The removal of the 5'-modification and the effect on product yield and detection | | |
|---|---|---|
| **Probe (pmol)** | **Yield (ng/ul)** | **Detection (mean pixel density)** |
| none | 10.8 | 0 |
| dA-VEEP1 (0.2) | 13.7 | 14.5 |
| dA-VEEP1 (1.0) | 11.9 | 22.9 |
| dA-VEEP6 (0.2) | 9.8 | 13.5 |
| dA-VEEP6 (1.0) | 10.2 | 17.1 |

### EXAMPLE 5

### Determination of Importance of 3' Termination

Example 5 demonstrates assay results using a 3' blocked detection probe and an unblocked probe.

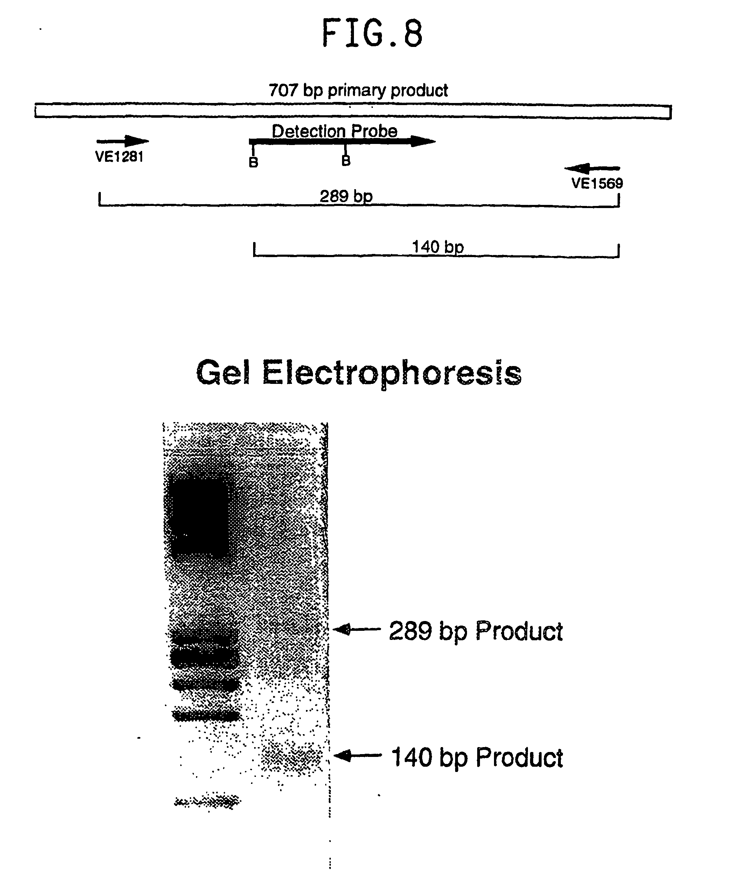

A biotinylated dA-VEEP1 probe was designed which lacked the 3' cordycepin and instead was terminated with a 3' hydroxyl group, capable of extending during the PCR reaction. The resultant non-specific products generated after amplification in the presence of this non-terminated probe included the typical 289 bp product and a 140 bp nested fragment. The 140 bp product was presumably the result of the amplification by the biotinylated, uncapped probe acting as a primer and the 3' primer, 1569R (Figure 8). As expected, the sample was detected in the lateral flow assay since all the fragments, including the nonspecific ones, would contain both ligands.

### EXAMPLE 6

### Multi-Analyte Detection Using a 3' Blocked Detection Probe to Detect Different Nucleic Acid Analyte Sequences

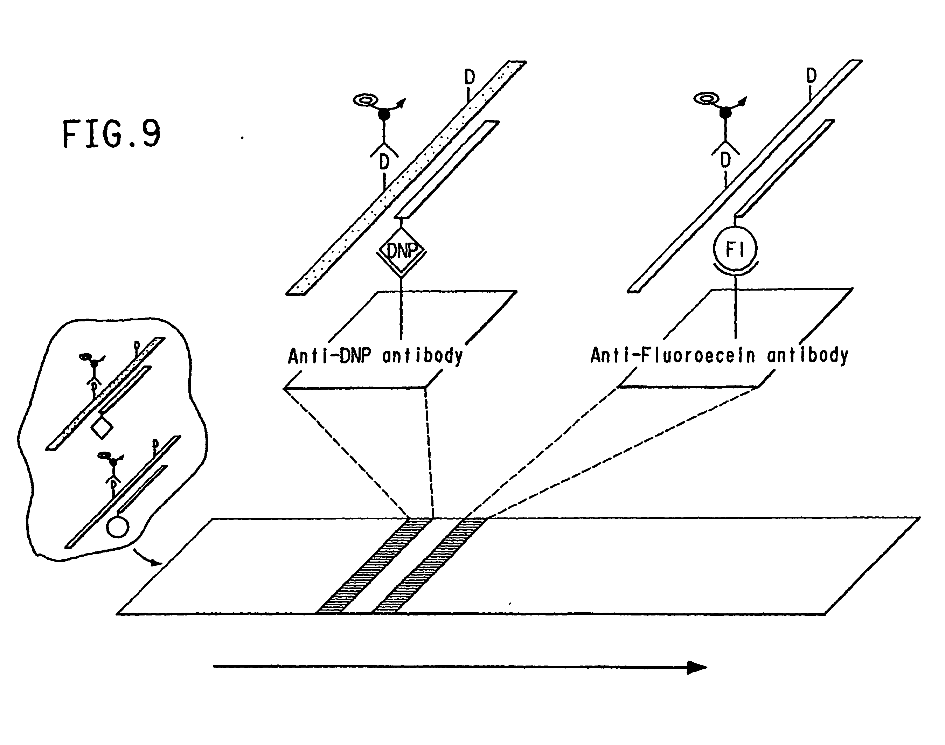

Example 6 demonstrates that the detection probe technology may be applied in a multianalyte fashion by incorporating different capture ligand moieties into the detection probes as depicted in Figure 9.

Two nucleic acid targets and gene-specific probes were synthesized using standard β-cyanoethyl phosphoramidite coupling chemistry as described above. The oligonucleotide targets. T97 and T84 were 97 and 84 bases long, respectively (Table 1). The 55 mer detection probe (dA-BP55.B3), specific for the T97 target, contained biotins as the ligand labels whereas the 42 mer probe (dA-BP42.D3), specific for the T84 target, contained dinitrophenol (DNP) groups. Targets were amplified in a multiplex fashion in the presence of 50 pmol each of T84 primers (TM1 and TM2), 100 pmol each of T97 primers (PL7 and PL8), 10 pmol each of the detection probes. 300 uM dNTPs with a 10% substitution of dTTP with digoxigenin-11-dUTP, 2.5 units of *Taq* polymerase in a final volume of 100 ul PCR buffer (50 mM KCl, 10 mM Tris-Cl, pH 8.4,1.5 mM MgCl₂, 0.01% gelatin). The DNA was amplified in a thermal cycler as follows: denaturation at 94°C for 1 min, annealing at 54°C for 1 min, and extension at 72°C for 1 min for 30 cycles followed by an extension at 72°C for 8 min. denaturation at 94°C for 2 min, with a final annealing step at 54°C for 15 min.

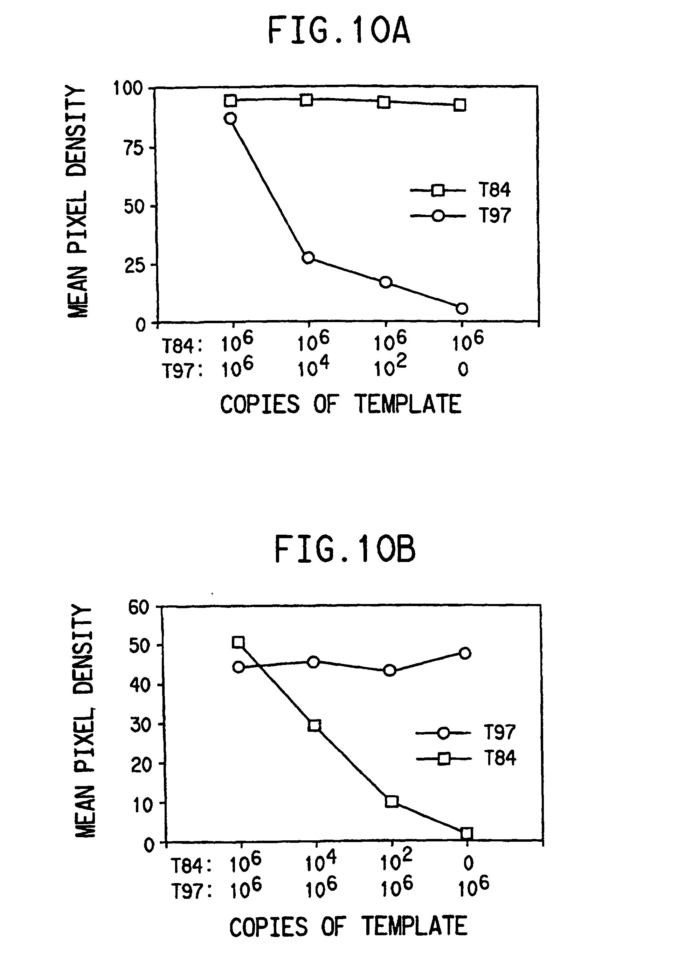

To test whether divergent target concentrations influenced amplification and detection, the initial concentration of one target was held constant while the concentration of the opposite target was serially diluted. The results (Figure 10) showed that both targets could be amplified in a multiplex fashion and detected by the lateral flow procedure by their respective membrane bound antibody (or streptavidin). Furthermore, the level of detection response, both on the gel and on the membrane strips, was indicative of the initial template concentration.

### EXAMPLE 7

### Application of the Detection Probe Technology to an Immuno-PCR Assay

Example 7 demonstrates the detection of hCG by combining the detection probe technology with the immuno-PCR assay procedure. In this procedure, an analyte is detected by a specific antibody bearing a DNA label. This DNA label is then amplified with sequence specific primers by standard PCR technology. The resultant product formed is indicative of the quantity of the analyte present. For in-depth discussions of the immuno-PCR technique, see Hendrickson, et al., (*NAR*, 23, 522, (1995)) and Sano, et al., (*Science,* 258, 120, (1992)).

### Reagents

The test analyte, human chorionic gonadotropin (hCG) was obtained from Calbiochem Corp. (La Jolla, CA). The murine monoclonal reporter antibody anti-hCG IgG1 (735329.306) that is used to covalently couple single-stranded (ss) DNA oligonucleotides to form the reporter conjugates was obtained from the DuPont Co. (Wilmington, DE). Murine monoclonal capture antibody anti-hCG IgG (735329.302) that is used for solid-phase capture was also obtained from the DuPont Co. (Wilmington, DE). Cross-linking reagents N-succinimidyl-S-acetylthioacetate (SATA) and sulfosuccinimidyl 4-(maleimidomethyl)cyclohexane-1-carboxylate (sulfo-SMCC) were purchased from Pierce Chemical Co.(Rockford, IL). PCR reagents and Taq DNA polymerase (AmpliTaq™) were obtained from Perkin-Elmer Corp.(Norwalk, CT). The §-cyanoethyl phosphoramidite amino-modifying reagent (Aminolink 2™)was purchased from Applied Biosystems (Foster City, CA).

### Oligonucleotide Synthesis

DNA oligonucleotide primers and reporter labels were prepared using standard *b*-cyanoethyl phosphoramidite coupling chemistry on controlled pore glass (CPG) supports in automated DNA oligonucleotide synthesizers (DuPont Generator™ (Wilmington, DE) or Applied Biosystems Model 392 (Foster City, CA)). The 5' terminus of the oligonucleotide label was derivatized using Aminolink 2™ to incorporate a primary aliphatic amine during the final coupling step of the synthesis. After the deprotection step, the DNA labels were ethanol precipitated. Additional purification steps to remove failure sequences from the preparation were not taken.

### Synthesis of the DNA oligonucleotide-antibody reporter conjugates

Synthesis of the DNA-labeled antibody conjugates was accomplished in four phases. In this approach. 5'amino-modified oligonucleotides and analyte-specific antibodies were independently activated by means of separate heterobifunctional crosslinking agents. The activated oligonucleotides and antibodies were then mixed to facilitate spontaneous coupling of the DNA label with the antibody. Specific conditions and protocols for each phase of the synthesis are described below:

### Preparation of acetylthioacetyl derivatized DNA

Amino-modified reporter oligonucleotide (T97, Table 1) was reacted with SATA as follows. An aliquot of the amino-modified oligonucleotide preparation, 50-60 nmoles, was added to 667 µL reaction mixture containing 100 mM sodium bicarbonate buffer (pH 9.0), 13.3 mg/mL SATA, and 50% dimethyl formamide (DMF). After 30 min at 25°C, the reaction mixture was immediately applied to a 1 x 20 cm Sephadex™ G-25 column (Pharmacia Biotech, Inc., Piscataway, NJ) and eluted at room temperature with 100 mM sodium phosphate buffer, pH 6.5, at a flow rate of ~1 mL/min. The absorbance of the effluent was monitored at 280 nm using a Pharmacia Model 2138 UVICORD S Monitor, and fractions were collected on a Pharmacia Model Frac-100 fraction collector (Pharmacia Biotech, Inc.. Piscataway, NJ). Two-milliliter fractions were collected, and those containing the acetylthioacetyl-modified oligonucleotides were pooled. These fractions were concentrated to a final volume of approximately 1.0 mL using Amicon Centricon® 3 concentrators (Amicon. Inc., Beverly, MA) and a Sorvall™ SM-24 rotor in a RC-5B centrifuge (Sorvall™, DuPont Co.. Wilmington, DE), spun at 7500 rpm (7000 x g) for 45 min at 20°C. The resulting samples were pooled, and further concentrated using the same procedure in a second set of Centricon® 3 concentrators. The acetylthioacetyl-modified oligonucleotide concentrate (approximately 1.0 mL) was recovered using the protocol recommended by the manufacturer (Amicon, Inc., Beverly, MA) and was saved at 20°C in the dark until it was needed for the final attachment of DNA label to reporter antibody.

### Preparation of Maleimide-Modified Antibodies

The reporter antibodies were derivatized with maleimide groups using sulfo-SMCC. An aliquot containing 25 nmoles of antibody was added to a reaction mixture (2.2 mL) containing 100 mM sodium phosphate buffer (pH 7.0), 1.2 mg/mL sulfo-SMCC, 1.5% DMF. (Note: The antibody modification reaction is started 75 min after beginning the preparation of the acetylthioacetyl-derivatized oligonucleotide. This timing is essential to minimize the deactivation of maleimide groups present in an aqueous solution, prior to the final conjugation reaction.) After the mixture had reacted for 30 min at 25°C, it was immediately applied to a 1 x 20 cm Sephadex™ G-25 column and eluted at room temperature with 100 mM sodium phosphate buffer, pH 6.5 at a flow rate of ~1 mL/min. The effluent was monitored and column fractions were collected as previously described for a Sephadex™ G-25 column. The first peak fractions (2.0 mL/fraction), which contained the maleimide-modified antibody, were pooled (4-6 mL) into one tube. The reaction product was ready for coupling to the modified oligonucleotides.

### DNA olieonucleotide-antibody conjugations

The pooled maleimide-modified antibody fraction was immediately added to a 15 mL Falcon® 2059 tube (Becton Dickinson, Cockeysville, MD). The concentrated acetylthioacetyl-modified oligonucleotides (approximately 1.0 mL) were added to the same tube and mixed well. The coupling reaction was initiated by adding 75 µL of 1 M hydroxylamine hydrochloride (Pierce Chemical Co., Rockford, IL), pH 7.0, 50 mM EDTA and mixing well. The reaction mixture was transferred to an Amicon Model 8010 mini-ultrafiltration stirred cell fitted with a YM30 membrane filter (Amicon, Inc., Beverly, MA). The cell was connected to a helium source adjusted to 60 psi. The coupling reaction proceeded with stirring at room temperature while the entire vessel was covered with aluminum foil to reduce exposure to light. The reaction mixture was concentrated to approximately 1.0 mL. removed from the MiniCell apparatus, and transferred to a 4.0 mL amber vial (Wheaton, Inc., Millville, NJ). This vial was incubated in the dark at room temperature on a Lab Quake™ tube rotator (Labindustries. Inc., Berkeley, CA) until the total reaction time reached 2 h. The reaction was terminated by the addition of 10 µL of 10 mM N-ethylmaleimide in DMF.

### Purification of the Oligonucleotide-Antibody Conjugates

The initial step in the purification of the conjugates used gel filtration high pressure liquid chromatography (HPLC). The HPLC system consisted of a Waters Model 600E multisolvent delivery system and Model-991 photodiode array detector (Milford, MA). Separation was accomplished using a mobile phase sodium phosphate buffer (200 mM. pH 7.0) at a flow rate of 1 mL/min through a 9.4 x 250 mm Zorbax® GF-250 column (MAC-MOD Analytical, Inc.. Chadds Ford, PA). Injections of the conjugate (200 µL) were made with a Waters 700 Satellite WISP automated injection system. The first HPLC peak fractions (0.3 mL/fraction) were mixtures of the oligonucleotide-antibody conjugate and the maleimide-modified antibody reaction component that were virtually free of the acetylthioacetyl-modified oligonucleotide precursor peak.

Spectrophotometric scans (320 nm to 220 nm) and A_{260/280} nm ratios measured on a Beckman DU 68 (Fullerton, CA) were used to determine which HPLC fractions contained the oligonucleotide-antibody conjugate. These results were confirmed by 3' end-labeling (terminal deoxynucleotidyl transferase and [a-³²P]-cordycepin, (3'dATP), NEN, DuPont, Boston, MA) of the conjugated oligonucleotide reporter labels followed by gel electrophoresis autoradiography. The conjugate-antibody peak fractions were pooled. The remaining unreacted, free oligonucleotides were removed from the pooled fractions using Microcon™ 100 microconcentrators (Amicon, Inc., Beverly, MA). The recovered conjugates were concentrated using the same procedure and then stored at 4°C.

To determine the purity and average DNA to antibody ratio for each of the conjugates, the conjugate concentrates were characterized by gel filtration HPLC using the conditions previously described. A single peak was observed, comprised of the conjugate and residual unconjugated antibody that was not removed during purification. The average DNA label to antibody ratios for each of the conjugate preparations were determined using the A_{260/280 nm} ratios obtained from absorbance values by the HPLC diode array detector.

### Immuno-PCR assay for single analytes:

### The immunoassay protocol

Capture antibody (6 µg/mL) in 100 mM sodium bicarbonate, pH 9.5, was immobilized on a 96-well, V-bottom, polycarbonate microtiter plate (Concord 25, MJ Research, Inc., Watertown. MA or Thermowell 961. Costar. Corp., Cambridge, MA) by adding 50 µL/well and incubating overnight (16 h) at 4°C or 1 h at room temperature. Antibody solutions were removed, and the wells were washed three times by adding the assay diluent/wash buffer. TBS/Tween (25 mM Tris, pH 7.4. 50 mM sodium chloride, 0.05% Tween-20), and immediately aspirating the buffer from the wells. The microtiter plate was inverted and slapped vigorously onto absorbent material to remove the residual wash buffer. Non-adsorbed sites in the microtiter wells were blocked with 200 µL/well of PBS-BLA buffer (10 mM sodium phosphate, pH 7.4, 150 mM sodium chloride, 2% BSA, 10% *b*-lactose, 0.02% sodium azide) and incubated for 1 h at room temperature. PBS-BLA buffer was removed, and the wells were washed three times as described.

Fifty-microliter aliquots made from serial dilutions of each test analyte were added to the wells of the microtiter plate containing the appropriate capture reagent. Negative control wells received 50 µL of TBS/Tween buffer. The microtiter plate was incubated at room temperature for 1 h. Analyte solutions were removed and the wells were washed three times as described. Fifty microliters of appropriately diluted oligonucleotide-antibody reporter conjugate were added to the test wells (a 1:500,000 dilution of the hCG DNA-labeled conjugate. The microtiter plate was incubated at room temperature for 1 h. Conjugate solutions were removed and the wells were washed three times as described.

### The PCR protocol

The microtiter plate was trimmed for insertion into the 96-well sample block of a Perkin-Elmer GeneAmp™ 9600 thermal cycler (Norwalk, CT). Amplification of the oligonucleotide label conjugated to the assay reporter antibody was performed using the polymerase chain reaction (PCR). The amplification reaction was done in a final volume of 50 µL containing 10 mM Tris-HCl (pH 8.3), 50 mM KCl, 1.5 mM MgCl₂, 200 µM dATP, 200 µM dCTP, 200 µM dGTP, 180 µM dTTP, 20-µM digoxigenin-11-dUTP, 50 nM each of PL7 and PL8 (Table 1)amplifying primers, 20 µM detection probe (da-BP55.B3) and 1.25 units Taq DNA polymerase (AmpliTaq™, Perkin-Elmer Corp., Norwalk, CT). Thirty microliters of sterile distilled water were added to each sample well of the microtiter plate. A 5 µL aliquot of the primer mix was added to the sample wells, followed by a 20 µL aliquot of liquid wax (Chill-out™. MJ Research Inc., Watertown, MA).

The microtiter plate was inserted into the thermal cycler sample block. The thermal cycler was ramped to 95°C for five min (initial denaturation step) and then held at 72°C for a hot start (15). A master mix (3.3X) containing the reaction buffer, sterile water, MgCl₂, and dNTPs was heated to 72°C and then the Taq DNA polymerase was added. A 15 µL aliquot of master mix at 72°C was added to each test well, dispensing below the liquid wax layer. The microtiter plate was covered and sealed with plate sealing tape (Costar, Inc., Cambridge, MA). A tray assembly was placed over top of the sealed microtiter plate. The tray assembly consisted of a plate weight milled (in-house) to fit inside the Perkin-Elmer MicroAmp™ tray. The heated cover of the thermal cycler was tightened in place to exert even pressure over the plate. Amplification was performed in 40 cycles using the following thermal cycling conditions: reaction volume set for 70 µL, 94°C for 10 s, 54°C for 15 s, and 72°C for 10 s. The final chain extension was made at 72°C for 45 s. The cycler was then ramped to 4°C and held until sample analysis.

### Detection and Analysis of PCR Products

The detection and quantitation of the immuno-PCR products are the same described in Example 1.

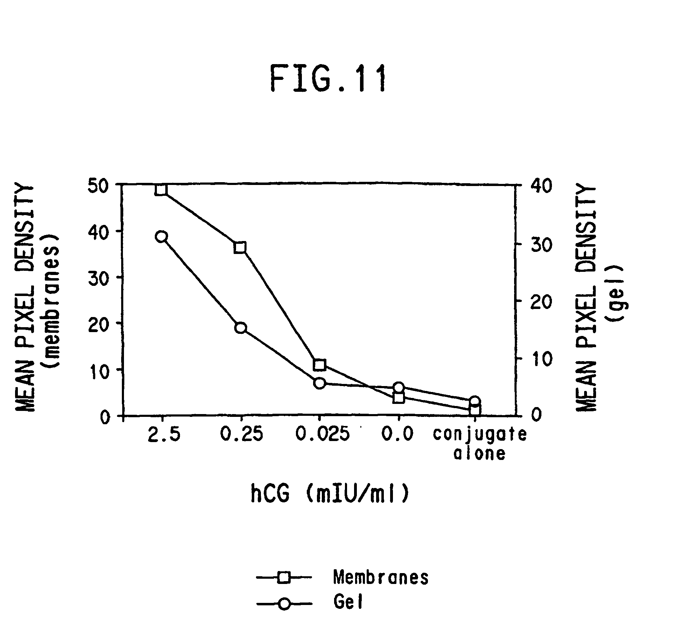

Experiments were performed using the replication inhibited probe technology in conjunction with the immuno-PCR procedure. The T97 antibody label, directed towards human chorionic gonadotropin (hCG), was amplified as described above in the presence of the biotinylated, sequence-specific detection probe (dA-BP55.B3) and the resultant product was detected by gel electrophoresis in the lateral flow format. The results showed that the detection probe technology can be successfully applied to the immuno-PCR procedure and that the signal generated on the membrane strips is representative of the amount of hCG present in the initial sample (Figure 11).

### EXAMPLE 8

### Demonstration of the use of the signal generating detection probe in the Homogenous Detection Probe System.

The homogeneous detection probe system (HDPS) is an application of the DP technology to a homogeneous detection format. Various concentrations of the target DNA would be amplified in the presence of 50 pmol each of primers, 10 pmol each of the SGDP subunits, and 200 uM dNTPs. Also included in the reaction mixture would be 1.5 units of Taq polymerase (Perkin-Elmer Corp., Norwalk, CT) in a final volume of 50 ul PCR buffer (50 mM KCl, 10 mM Tris-Cl, pH 8.4. 1.5 mM MgCl₂, 0.01% gelatin). The DNA would be amplified in a thermal cycler (Perkin-Elmer Corp.) as follows: denaturation at 94°C for 1 min, annealing at 55°C for 1 min, and extension at 72°C for 1 min for 35 cycles followed by an extension at 72°C for 5 min. After PCR, the fluorescence of the product could be measured in a fluorometer. The amount of fluorescence would be indicative of the amount of product formed since the smaller quenching subunit would be displaced from the larger fluorescing subunit which hybridizes to the accumulating product. Not only would the fluorescence response be indicative of the amount of product formed at the end of the PCR cycling, but the response could be measured during the cycling for real-time measurement of product formation.

Initial experiments for the Homogenous Detection Probe System (HDPS) were performed to ascertain whether the DABCYL labeled oligonucleotide (SQDP) quenched the fluorescent signal of the EDANS labeled oligonucleotide (SGDP) when the two subunits were hybridized to each other. Samples below were prepared in 50 ul PCR buffer (50 mM KCl, 10 mM Tris-Cl, pH 8.4. 1.5 mM MgCl₂, 0.01% gelatin). Under ultraviolet excitation (336 nm), 600 pmol of the short oligonucleotide subunit that contained the quenching reagent produced relatively little emission (Intensity = 1.9) at 490 nm whereas 300 pmol of the SGDP containing the fluorescing dye produced an emission at 490 nm (Intensity = 42.5 nm). When the two labeled oligonucleotides were hybridized together at room temperature, the fluorescence was quenched (Intensity = 11.0). When a noncomplementary, DABCYL-labeled oligonucleotide was added to the above EDANS oligonucleotide (where no hybridization (and quenching) should take place), a 490 nm emission was detected (Intensity = 38.5).

## Claims

1. A method for the detection of a target nucleic acid analyte sequence in a nucleic acid replication reaction, comprising the steps of:
(i) contacting at least one target nucleic acid sequence with a nucleic acid replication composition containing a homogeneous detection probe system comprising at least one pair of nucleic acid probes said pair consisting of:
(a) a first, signal generating probe comprising a first member of a reporter pair, a target domain, a first probe binding domain and replication inhibitor moiety wherein said inhibitor moiety is a 3'deoxynucleotide, and;
(b) a second, signal modifying probe comprising a second member of a reporter pair and a second probe binding domain complementary to said first probe binding domain wherein the first and second members of the reporter pair are capable of reacting with each other to produce a detectable signal;
(ii) replicating the target nucleic acid sequence in the replication composition of step (i) to produce a nucleic acid analyte and under reaction conditions that permit the formation of an analyte/probe hybrid wherein said hybrid consists of said target analyte nucleic acid and the signal generating detection probe; and
(iii) detecting the presence said analyte/probe hybrid.

2. The method of claim 1 wherein the 3' deoxynucleotides is cordycepin.

3. The method of claim 1 wherein said reporter pair is selected from the group consisting of fluorophores and enzymes.

4. A homogenous detection probe system comprising at least one pair of nucleic acid probes consisting of a first signal generating probe comprising a first member of a reporter pair, a target domain, a first probe binding domain and replication inhibitor moiety, wherein said inhibitor moiety is a 3'deoxynucleotide, and a second signal modifying probe comprising a second member of a reporter pair and a second probe binding domain complementary to said first probe binding domain wherein the first and second members of the reporter pair are capable of reacting with each other to produce a detectable signal.

## Patentansprüche

1. Verfahren zum Nachweis einer Target-Nukleinsäureanalytsequenz in einer Nukleinsäure-Replikationsreaktion, umfassend die Schritte von:
(i) Kontaktieren von mindestens einer Target-Nukleinsäuresequenz mit einer Nukleinsäure-Replikationszusammensetzung, enthaltend ein homogenes Detektionssondensystem, das mindestens ein Nukleinsäuresonden-Paar umfasst, wobei das Paar aus Folgendem besteht:
(a) einer ersten Signal-generierenden Sonde, umfassend ein erstes Glied eines Reporterpaars, eine Targetdomäne, eine erste Sonden-bindende Domäne und Replikationsinhibitorkomponente, worin die Inhibitorkomponente ein 3'-Desoxynukleotid darstellt, und
(b) einer zweiten Signal-modifizierenden Sonde, umfassend ein zweites Glied eines Reporterpaars und eine zweite Sonden-bindende Domäne, die zur ersten Sonden-bindenden Domäne komplementär ist, worin die ersten und zweiten Glieder des Reporterpaars zur Reaktion miteinander zur Produktion eines nachweisbaren Signals fähig sind;
(ii) Replizieren der Target-Nukleinsäuresequenz in der Replikationszusammensetzung von Schritt (i) zur Produktion eines Nukleinsäureanalyts und unter Reaktionsbedingungen, die die Bildung einer Analyt-/Sondenhybride erlauben, worin die Hybride aus dem Target-Nukleinsäureanalyten und der Signal-generierenden Detektionssonde besteht; und
(iii) Detektion des Vorliegens der Analyt/Sonden-Hybride.

2. Verfahren nach Anspruch 1, worin das 3'-Desoxynukleotid Cordycepin darstellt.

3. Verfahren nach Anspruch 1, worin das Reporteipaar aus der Gruppe ausgewählt ist, bestehend aus Fluorophoren und Enzymen.

4. Homogenes Detektionssondensystem, umfassend mindestens ein Nukleinsäuresonden-Paar, das aus einer ersten Signal-generierenden Sonde besteht, umfassend ein erstes Glied eines Reporterpaars, eine Targetdomäne, eine erste Sonden-bindende Domäne und Replikationsinhibitorkomponente, worin die Inhibitorkomponente ein 3'-Desoxynukleotid darstellt und eine zweite Signalmodifizierende Sonde, umfassend ein zweites Glied eines Reporterpaars und eine zweite Sonden-bindende Domäne, die zur ersten Sonden-bindenden Domäne komplementär ist, worin die ersten und zweiten Glieder des Reporterpaars zur Reaktion miteinander zur Produktion eines nachweisbaren Signals fähig sind.

## Revendications

1. Procédé de détection d'une séquence d'analyte d'acide nucléique cible dans une réaction de réplication d'acide nucléique, comprenant les étapes consistant à:
(i) mettre en contact au moins une séquence d'acide nucléique cible avec une composition de réplication d'acide nucléique contenant un système de sondes de détection homogène comprenant au moins une paire de sondes d'acide nucléique, ladite paire consistant en:
(a) une première sonde génératrice de signal comprenant un premier membre d'une paire de rapporteurs, un domaine cible, un premier domaine de liaison de sonde et un fragment inhibiteur de réplication, où ledit fragment inhibiteur est un 3'-désoxynucléotide, et
(b) une deuxième sonde modificatrice de signal comprenant un deuxième membre d'une paire de rapporteurs et un deuxième domaine de liaison de sonde complémentaire audit premier domaine de liaison de sonde, où lesdits premier et deuxième membres de la paire de rapporteurs sont capables de réagir l'un avec l'autre pour produire un signal détectable;
(ii) répliquer la séquence d'acide nucléique cible dans la composition de réplication de l'étape (i) pour produire un analyte d'acide nucléique et dans des conditions de réaction qui permettent la formation d'un hybride analyte/sonde, où ledit hybride consiste en ledit analyte d'acide nucléique cible et en la sonde de détection génératrice de signal; et
(iii) détecter la présence dudit hybride analyte/sonde.

2. Le procédé de la revendication 1, dans lequel le 3'-désoxynucléotide est de la cordycépine.

3. Le procédé de la revendication 1, dans lequel ladite paire de rapporteurs est sélectionnée parmi le groupe consistant en fluorophores et en enzymes.

4. Système de sondes de détection homogène comprenant au moins une paire de sondes d'acide nucléique consistant en une première sonde génératrice de signal comprenant un premier membre d'une paire de rapporteurs, un domaine cible, un premier domaine de liaison de sonde et un fragment inhibiteur de réplication, où ledit fragment inhibiteur est un 3'-désoxynucléotide, et une deuxième sonde modificatrice de signal comprenant un deuxième membre d'une paire de rapporteurs et un deuxième domaine de liaison de sonde complémentaire audit premier domaine de liaison de sonde, où les premier et deuxième membres de la paire de rapporteurs sont capables de réagir l'un avec l'autre pour produire un signal détectable.
